(19) Europäisches Patentamt
European Patent Office
Office européen des brevets

(11) **EP 4 124 398 B1**

(12) **EUROPÄISCHE PATENTSCHRIFT**

(45) Veröffentlichungstag und Bekanntmachung des Hinweises auf die Patenterteilung:
**10.04.2024  Patentblatt 2024/15**

(21) Anmeldenummer: **21187849.1**

(22) Anmeldetag: **27.07.2021**

(51) Internationale Patentklassifikation (IPC):
**B21B 37/00** (2006.01)

(52) Gemeinsame Patentklassifikation (CPC):
**B21B 37/00; G06N 7/01; G06N 20/00;** G01N 33/20

(54) **VERFAHREN ZUR BESTIMMUNG MECHANISCHER EIGENSCHAFTEN EINES WALZGUTES MIT HILFE EINES HYBRIDEN MODELLS**

METHOD FOR DETERMINING MECHANICAL PROPERTIES OF A PRODUCT TO BE ROLLED USING A HYBRID MODEL

PROCÉDÉ DE DÉTERMINATION DES PROPRIÉTÉS MÉCANIQUES D'UN PRODUIT LAMINÉ A L'AIDE D'UN MODÈLE HYBRIDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**01.02.2023  Patentblatt 2023/05**

(73) Patentinhaber: **Primetals Technologies Austria GmbH**
**4031 Linz (AT)**

(72) Erfinder:
- **Nguyen Duy, Du**
  **4232 Hagenberg im Mühlkreis (AT)**
- **Astleithner, Katharina**
  **4284 Tragwein (AT)**
- **Bragin, Sergey**
  **4030 Linz (AT)**

- **Jax, Klaus**
  **4202 Hellmonsödt (AT)**
- **Rimnac, Axel**
  **4020 Linz (AT)**
- **Seyr, Alfred**
  **3125 Statzendorf (AT)**
- **Strasser, Sonja**
  **4451 Garsten (AT)**

(74) Vertreter: **Metals@Linz**
**Primetals Technologies Austria GmbH**
**Intellectual Property Upstream IP UP**
**Turmstraße 44**
**4031 Linz (AT)**

(56) Entgegenhaltungen:
**WO-A1-98/18970          JP-A- 2010 172 962**
**JP-A- 2011 220 708**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**EP 4 124 398 B1**

**Beschreibung**

Die Erfindung betrifft ein Verfahren zur Bestimmung mechanischer Eigenschaften eines Walzgutes mit Hilfe eines hybriden Modells, umfassend ein physikalisches Produktionsmodell und ein statistisches Datenmodell. Weiterhin betrifft die Erfindung ein Verfahren zur Erstellung eines hybriden Modells, umfassend ein physikalisches Produktionsmodell und ein statistisches Datenmodell.

In einer Walzanlage wird ein metallisches Walzgut, beispielsweise ein Stahlband, gewalzt, um seine Dicke zu reduzieren. Weiterhin wird das Walzgut während und nach dem Walzprozess in der Regel mehreren Abkühlungsvorgängen unterzogen, wobei die Kühlung aktiv, beispielsweise durch Aufbringen von Kühlflüssigkeit, oder passiv durch Wärmeabstrahlung an die Umgebung oder Wärmeleitung an ein umgebendes Medium (Luft bzw. Transportrollen) erfolgen kann. Sowohl die Dickenreduktion des Walzgutes in den einzelnen Walzstichen beim Durchgang durch die Walzanlage als auch der zeitliche Temperaturverlauf des Walzgutes dabei sowie in einem anschließenden Abkühlprozess beeinflusst maßgeblich die mechanischen Eigenschaften wie Zugfestigkeit $Y_s$, Streckgrenze $T_s$ oder Bruchdehnung $A_l$ des produzierten Walzgutes. Um daher sicherzustellen, dass die mechanischen Eigenschaften eines produzierten Walzgutes bestimmte vorgegebene Werte aufweist, ist in der Regel eine Beprobung, d.h. eine Entnahme eines Probenstücks von dem betreffenden Walzgut und eine Messung dessen mechanischer Eigenschaften notwendig.

Die Beprobung von Walzgut, beispielsweise von Stahlband, ist entsprechend aufwändig, weswegen versucht wird, diese weitgehend zu vermeiden und stattdessen durch eine Berechnung der mechanischen Eigenschaften zu ersetzen. Dafür werden gemäß dem Stand der Technik physikalische bzw. metallurgische Modelle herangezogen, mit denen die zeitliche Entwicklung verschiedener Materialeigenschaften eines Walzgutes während des Produktionsprozesses rechnerisch simuliert und aus denen die gesuchten mechanischen Eigenschaften abgeleitet werden können.

Beispielsweise ist aus der CN101046682 (A) ein Verfahren bekannt, bei dem der Temperaturverlauf von warmgewalzten Metallbändern während deren Produktion in einer Vorwalzstraße, in einer Fertigwalzstraße und in einer Kühlstrecke modelliert wird sowie metallurgische Eigenschaften der Metallbänder ermittelt werden, woraus anhand empirischer Formeln die Zugfestigkeit $Y_s$, die Streckgrenze $T_s$ bzw. die Bruchdehnung $A_l$ der Metallbänder ermittelt werden.

Weiterhin wird mit Hilfe von sogenannten statistischen Datenmodellen versucht, Korrelationen zwischen beispielsweise der chemischen Zusammensetzung und/oder während der Produktion eines Walzgutes erfassten Prozessparametern einerseits und den mechanischen Eigenschaften des Walzgutes andererseits zu ermitteln. Die chemische Zusammensetzung bzw. die Prozessparameter bilden in diesem Fall die sogenannten Eingangsgrößen eines statistischen Modells, während die mechanischen Eigenschaften dessen sogenannte Ausgangsgrößen darstellen.

Dabei wird in der Regel für eine Produktionsanlage ein entsprechendes statistisches Datenmodell erstellt, indem für bestimmte, an der Anlage produzierte Walzprodukte deren Eingangsgrößen mit den in jeweiligen Beprobungen erfassten Ausgangsgrößen in Beziehung gesetzt werden, was auch als 'Trainieren' des statistischen Datenmodells bzw. 'maschinelles Lernen' bezeichnet wird. Die Eingangs- und Ausgangsgrößen jener Walzprodukte, die zum Trainieren des statistischen Datenmodells verwendet werden, werden auch als sogenannte 'Trainingsdatensätze' bezeichnet. Die Gesamtheit der Ausgangsgrößen der Testdatensätze werden auch als 'Lösungsraum' des statistischen Datenmodells bezeichnet.

Zur Überprüfung der Aussagekraft des trainierten statistischen Datenmodells wird dieses in der Regel validiert, indem weitere, an derselben Anlage produzierte Walzprodukte einer Beprobung unterzogen werden und die dabei ermittelten Werte als sogenannte 'Zielgrößen' mit den Ausgangsgrößen (entsprechend den Vorhersagewerten) des trainierten statistischen Datenmodells verglichen werden. Die zur Validierung des statistischen Datenmodells verwendeten Eingangs- und Zielgrößen der weiteren Walzprodukte werden als sogenannte 'Testdatensätze' bezeichnet.

Mit einem derart trainierten und validierten statistischen Datenmodell lassen sich in der Folge aus den Eingangsgrößen (z.B. chemische Zusammensetzung, Produktionsparameter) von weiteren, an derselben Anlage produzierten Walzprodukten deren Ausgangsgrößen (mechanische Eigenschaften) ohne entsprechende physische Beprobung ermitteln. Statistische Datenmodelle können beispielsweise als künstliche neuronale Netzwerke oder als Random-Forest-Modelle ausgebildet sein.

Aus der CN107609647 (A) ist ein Verfahren zur Erstellung eines statistischen Datenmodells zur Vorhersage mechanischer Eigenschaften von Walzprodukten aus unterschiedlichen Legierungsbestandteilen und unterschiedlichen Wärmebehandlungen auf Basis eines neuronalen Netzwerks bekannt, wobei die mechanischen Eigenschaften für eine Anzahl von Walzprodukten mit Hilfe eines mechanischen Testverfahrens in Form von Trainingsdatensätzen und Testdatensätzen ermittelt werden, und wobei das neuronale Netzwerk mit Hilfe der Trainingsdatensätze trainiert und anhand der Testdatensätze evaluiert wird.

Die JP 2005-315703 (A) offenbart ein Verfahren, in dem für warmgewalzte Stahlbänder zunächst mit Hilfe eines metallurgischen Modells metallurgische Eigenschaften, beispielsweise die sich während des Produktionsprozesses ausbildenden metallurgischen Phasenanteile und Korngrößen, ermittelt und zusammen mit der chemischen Zusammensetzung und den Produktionsparametern der Metallprodukte einem neuronalen Netzwerk als Eingangsgrößen zugeführt, mit dessen Hilfe anschließend mechanische Eigenschaften der Metallprodukte, wie Zugfestigkeit $Y_s$, Streckgrenze $T_s$ oder Bruchdehnung $A_l$ und duktil-spröde Übergangstemperatur, ermittelt werden.

Die US 2018/260717 A1 offenbart ein Verfahren zur Vorhersage mechanischer Eigenschaften von mikrolegiertem Stahl, wobei in einem ersten Schritt anhand eines Random-Forest-Modells unabhängige Einflussfaktoren der mechanischen Eigenschaften ermittelt werden, in einem zweiten Schritt die Ausbildung von Karbonitriden auf Basis eines thermodynamischen Phasenmodells während des Walzprozesses modelliert werden, in einem dritten Schritt die mechanischen Eigenschaften als Summe einzelner empirisch-metallurgischer Teilmodelle der unabhängigen Variablen dargestellt werden, in einem vierten Schritt die Teilmodelle mittels eines Backpropagation-Algorithmus parametriert werden und in einem fünften Schritt die Teilmodelle anhand von Plausibilitätsüberlegungen und Produktionsdaten verifiziert werden.

Ein statistisches Datenmodell kann prinzipiell nur aus solchen Eingangsgrößen verlässliche Ausgangsgrößen ermitteln, die mit akzeptabler Genauigkeit und Reproduzierbarkeit erfasst werden können. Daher weisen die aus dem Stand der Technik bekannten Verfahren, die statistische Datenmodelle verwenden, Nachteile auf, da sie manche für die gesuchten mechanischen Eigenschaften von Walzgut relevante Prozessparameter während dessen Produktion nicht berücksichtigen: in einer Kühlstrecke einer Warmwalzanlage beispielsweise können Wasserdampfbildung und Wasserlacken am Walzgut eine Temperaturmessung empfindlich stören, sodass fehlende bzw. ungültige Messwerte durch Schätzwerte ersetzt werden müssen. In der Folge ist eine valide Erfassung der entsprechenden Kühlrate als Eingangsgröße für ein statistisches Datenmodell ohne Modellierung des Produktionsprozesses nicht möglich, wobei die Kühlrate jedoch ein wichtiger Einflussfaktor für die Ausprägung des metallurgischen Gefüges und in Folge der mechanischen Eigenschaften des Walzgutes ist.

Die JP 2011 220708 A zeigt ein Verfahren zur Bestimmung mechanischer Eigenschaften eines in einem Walzwerk produzierten ersten Walzguts mit Hilfe eines hybriden Modells, wobei ein neuronales Netzwerk zur Berechnung verwendet wird.

Nachteilig an den aus dem Stand der Technik bekannten statistischen Datenmodellen ist zudem, dass die Gültigkeit der vorhergesagten Ausgangsgrößen streng genommen auf den Ort der Probennahme beschränkt ist. Da eine Probenentnahme in der Regel jedoch nur an einer Stelle des Walzgutes erfolgt, werden die Auswirkungen von produktionsbedingten Schwankungen der Prozessparameter über die Länge eines Walzgutes nicht erfasst. Insbesonders bei zu Coils aufgewickelten Walzbändern erfolgt die Probennahme aus Gründen der Zugänglichkeit nahe deren jeweiligem Bandende.

Weiterhin kann ein statistisches Datenmodell nur innerhalb seines Lösungsraumes valide Aussagen treffen, während außerhalb dieses Lösungsraumes die Ergebnisse nicht vertrauenswürdig sind. Auch in schwach besetzten Bereichen der Eingangsgrößen eines statistischen Datenmodells, in denen nur eine geringe Zahl von Trainingsdatensätzen vorhanden ist, ist die Genauigkeit der Ergebnisse beschränkt. Anders ausgedrückt bedeutet dies, dass ein statistisches Datenmodell nur in jenen Bereichen der Eingangsgrößen valide Aussagen machen kann, in dem beim Trainieren eine ausreichende Anzahl von Datensätzen in entsprechender Qualität vorliegen, sodass ein statistisches Datenmodell keine valide Aussage treffen kann, wenn ein Prozessdatensatz eines Walzguts, dessen mechanische Eigenschaften vorhergesagt werden sollen, weit außerhalb des Bereichs liegt, der von den Eingangsgrößen der Trainingsdatensätze des betreffenden statistischen Datenmodells aufgespannt wird.

Aus den genannten Gründen ist die Extrapolationsfähigkeit von statistischen Datenmodellen in der Praxis gering, da die zur Verfügung stehenden Datensätze zum Trainieren der Modelle zwar umfangreich sind, jedoch nur eine bestimmte Anzahl an Walzgutsorten beinhalten und nur bestimmte Fahrweisen der Produktion auf einer spezifischen Anlage abbilden. Auch gerundete Messwerte in den Trainingsdatensätzen können zusätzliche Probleme verursachen.

Der Erfindung liegt die Aufgabe zugrunde, die Nachteile der aus dem Stand der Technik bekannten Verfahren zu überwinden.

Die Aufgabe wird erfindungsgemäß durch ein Verfahren zur Bestimmung mechanischer Eigenschaften eines Walzgutes mit Hilfe eines hybriden Modells, das hybride Modell umfassend ein physikalisches Produktionsmodell und ein statistisches Datenmodell, mit den Merkmalen des Anspruchs 1 gelöst. Weiterhin wird die Aufgabe durch ein Verfahren zur Erstellung eines hybriden Modells mit den Merkmalen des Anspruchs 9 gelöst. Vorteilhafte Ausgestaltungen der Erfindung sind Gegenstand der Unteransprüche.

Bei dem erfindungsgemäßen Verfahren zur Bestimmung mechanischer Eigenschaften eines in einem Walzwerk produzierten Walzgutes werden, ausgehend von einem Produktionsdatensatz $P_f$ eines ersten Walzguts, mechanische Eigenschaften $J_f$ des ersten Walzguts mit Hilfe eines hybriden Modells bestimmt. Bei dem Walzwerk kann es sich insbesondere um ein Warmwalzwerk handeln.

Das hybride Modell umfasst Produktionsdatensätze $P_i$ weiterer in dem Walzwerk produzierter Walzgüter, ein physikalisches Produktionsmodell und ein trainiertes und validiertes statistisches Datenmodell. Die weiteren Walzgüter bilden eine erste Menge $I_1$ an in dem Walzwerk produzierten Walzgütern. Zu jedem der Produktionsdatensätze $P_i$ der weiteren Walzgüter liegt jeweils ein korrespondierender Beprobungsdatensatz $B_i$ vor, der die nach der Produktion des betreffenden weiteren Walzguts in dem Walzwerk mittels einer Beprobungsvorrichtung ermittelten mechanischen Eigenschaften des weiteren Walzguts umfasst. Die Beprobungsdatensätze $B_i$ der weiteren Walzgüter sind selbst jedoch nicht Teil des hybriden Modells sondern werden lediglich zu dessen Erstellung herangezogen. Kurz ausgedrückt bedeutet dies, dass sämtliche weiteren, in dem Walzwerk produzierten Walzguter beprobt und deren mechanische Eigenschaften dabei erfasst wurden.

Die Produktionsdatensätze $P_i$ der weiteren Walzgüter sind dem statistischen Datenmodell bereits als Eingangsgrößen im Zuge von dessen Erstellung in Form von Trainingsdatensätzen in oben definiertem Sinn zugeführt worden. Nach der Validierung werden die Vorhersageeigenschaften des statistischen Datenmodells nach dessen letztmaligem 'Trainieren' nicht mehr geändert.

Die Daten des Produktionsdatensatzes $P_f$ des ersten Walzguts bzw. der Produktionsdatensätze $P_i$ der weiteren Walzgüter werden jeweils bei - bzw. zeitlich gesehen während - der Produktion des jeweiligen Walzguts in dem Walzwerk erfasst. Dementsprechend können einzelne oder alle Daten eines Produktionsdatensatzes zeitabhängig sein, in welchem Fall die entsprechenden zeitabhängigen Daten jeweils mehrere Datenwerte von verschiedenen Zeitpunkten während des Produktionsprozesses umfassen.

Ein Produktionsdatensatz $P_f$ des ersten bzw. der weiteren Walzgüter $P_i$ umfasst jeweils nur solche Daten, die entweder messtechnisch erfassbar sind (beispielsweise aus einer Temperaturmessung oder einer Geschwindigkeitsmessung des betreffenden Walzguts) oder die automationstechnisch oder von einem Bediener vorgegeben werden können. Es handelt sich dabei somit um Stell- oder Messgrößen, die beispielsweise von einer Anlagenautomatisierung vor bzw. während der Produktion der entsprechenden Walzgüter in dem Walzwerk datentechnisch erfasst und gespeichert werden können und dabei Daten umfassen können, die bereits vor Produktionsstart des betreffenden Walzguts bekannt sind: beispielsweise können Fremdelementanteile, Setupwerte für Aggregate des Walzwerke oder eine Ausgangsdicke eines Walzguts beim Eintritt in das Walzwerk von einem übergeordneten Leitsystem oder manuell von einem Bediener vorgegeben werden. Anders ausgedrückt umfassen die Produktionsdatensatzes $P_f$ des ersten Walzguts bzw. der Produktionsdatensätze $P_i$ Daten, die auf den Produktionsvorgang des betreffenden Walzguts Einfluss nehmen.

Das physikalische Produktionsmodell zur Modellierung des Produktionsprozesses eines in dem Walzwerk produzierten Walzguts umfasst einen Satz von metallurgischen Modellparametern, die im Weiteren unter dem Symbol y subsummiert werden. Diese metallurgischen Modellparameter y stellen im Rahmen der Erfindung physikalische Einflussgrößen dar, deren Werte innerhalb plausibler Grenzen gewählt werden und mit deren Hilfe die Modellierung des Produktionsprozesses in guter Näherung zum real ablaufenden Produktionsprozess ausgeführt werden kann.

Mit Hilfe des physikalischen Produktionsmodells mit den metallurgischen Modellparametern $\gamma$ ist aus dem Produktionsdatensatz $P_f$ des ersten Walzguts ein erster mechanischer Datensatz $C_f$ des ersten Walzguts, ein weiterer Produktionsdatensatz $F_f$ des ersten Walzguts, umfassend weitere Produktionsdaten des ersten Walzguts während dessen Produktion in dem Walzwerk, und ein metallurgischer Datensatz $K_f$ des ersten Walzguts, umfassend metallurgische Eigenschaften des ersten Walzguts während dessen Produktion in dem Walzwerk, ermittelbar. Dabei bezieht sich der Ausdruck 'während der Produktion' auf eine Zeitspanne, die zwischen dem Produktionsstart und dem Abschluss des Produktionsvorganges des ersten Walzguts an der Walzanlage liegt.

Die Ermittlung des ersten mechanischen Datensatzes $C_f$, des weiteren Produktionsdatensatzes $F_f$ und des metallurgischen Datensatzes $K_f$ erfolgt dabei in Form einer Modellierung, d.h. der Produktionsprozess des ersten Walzguts wird anhand des physikalischen Produktionsmodells auf Basis von physikalischen Gleichungen und auf Basis empirischer Gleichungen unter Zuhilfenahme der metallurgischen Modellparameter $\gamma$ rechnerisch nachgebildet, sodass die Daten mechanischen Datensatzes $C_f$, des weiteren Produktionsdatensatzes $F_f$ und des metallurgischen Datensatzes $K_f$ in mathematischer Hinsicht jeweils explizit von den metallurgischen Modellparametern y abhängen.

Der erste mechanische Datensatz $C_f$ umfasst die mechanischen Eigenschaften des ersten Walzguts nach dessen Produktion in dem Walzwerk. Dabei bezieht sich der Ausdruck 'nach der Produktion' auf einen Zeitraum, in dem die Produktion des betreffenden Walzguts in dem Walzwerk abgeschlossen ist und weitere Verarbeitungsschritte (beispielsweise eine Beprobung des Walzguts oder ein Transport an eine weiterverarbeitende Anlage) erfolgen können.

Als physikalische Gleichungen des physikalischen Produktionsmodells kann beispielsweise eine Wärmeleitungsgleichung unter Berücksichtigung entsprechender Randbedingungen und in Verbindung mit einem gekoppelten Gleichungssystem für die Phasenanteile eines Walzguts herangezogen werden.

Beispielsweise sind - unter besonderer Berücksichtigung von Kohlenstoffdiffusion zwischen einzelnen metallurgischen Phasen - derartige Gleichungen aus Wolff et al., Archives of Mechanics, 59, 4-5, S.435-466, 2007 bekannt: darin stellt (2.27) eine Wärmeleitungsgleichung für Stahl dar, in der neben der austenitischen Phase weitere metallurgische Phasen und die freigesetzte latente Wärme beim Übergang der austenitischen Phase in die weiteren Phasen berücksichtigt wird. Weiterhin repräsentiert (2.41) ein gekoppeltes Gleichungssystem für die Umwandlung der einzelnen metallurgischen Phasen ineinander, in das die Übergangsraten zwischen einzelnen Phasen als metallurgische Modellparameter eingehen. Die Ausdrücke (2.30) und (2.31) stellen die Anfangsbedingung bzw. die Randbedingung für die Wärmeleitungsgleichung dar. Die spezifische Wärmekapazität und die Wärmeleitfähigkeit des Stahls sowie der Wärmeleitkoeffizient der Randbedingung hängen dabei jeweils vom Anteil der einzelnen metallurgischen Phasen im Stahl ab.

Eine alternative Form der Wärmeleitungsgleichung lautet

$$\frac{\partial}{\partial t}\sum_{k=1}^{m}p_k h_k - \frac{\partial}{\partial x}\left[\frac{\lambda(h,p_1,\ldots,p_m)}{\rho}\frac{\partial T(h,p_1,\ldots,p_m)}{\partial x}\right] = s \qquad (1)$$

In Gleichung (1) sind $p_k$ mit $k=1,\ldots,m$ Phasenanteile des Walzguts, beispielsweise ein Austenitanteil, ein Ferritanteil, ein Zementitanteil und/oder weitere Anteile. Die Phasenanteile sind stets nicht negativ und summieren sich zu Eins auf. Die Wärmeleitfähigkeit $\lambda$ ist hierbei ebenfalls als eine von der der Enthalpiedichte h und den Phasenanteilen $p_k$ abhängige Funktion ausgedrückt. Die Größe $\rho$ bezeichnet die für alle Phasenanteile gleich angenommene Dichte des Walzguts. Die Größe s bezeichnet einen beim Durchtritt des Walzguts durch einen Walzspalt entstehenden Wärmequellterm, der proportional zur Fließspannung des im Walzspalt umgeformten Walzstücks ist. Der Wärmequellterm kann bei Kenntnis der Walzspaltgeometrie und der Walzgeschwindigkeit in bekannter Weise ermittelt werden.

Die Größe h ist eine Enthalpiedichte des betrachteten Walzguts, wobei

$$h = \sum_{k=1}^{m}p_k h_k \qquad (2)$$

gilt. Weiterhin existieren für jeden Phasenanteil $p_k$ bekannte Abhängigkeiten zwischen dem Enthalpiedichteanteil $h_k$ des jeweiligen Phasenanteils und der zugehörigen Temperatur $T_k$, d.h. die Temperatur $T_k = T_k(h_k)$ ist eine streng monoton steigende Funktion des Enthalpiedichteanteils $h_k$. Dabei gilt der Zusammenhang

$$T_1(h_1) = T_2(h_2) = \cdots = T_m(h_m) = T \qquad (3)$$

da die Temperatur an einer Stelle nur einen einzigen Wert annehmen kann, der für alle Phasenanteile $p_k$ derselbe ist. Für die zeitliche Änderung der Phasenanteile $p_k$ (Phasenumwandlungsgleichungen) lässt sich wiederum ein gekoppeltes Differentialgleichungssystem der Form

$$\frac{\mathrm{d}p_k}{\mathrm{d}t} = f_{\mathrm{p}k}(T, p_1, \ldots, p_m), \; k = 1, \ldots, m \qquad (4)$$

mit Modellfunktionen $f_{pk}(T, p_1,\ldots,p_m)$ darstellen, die die Umwandlungs- bzw. Wachstumskinetik einzelner metallurgischer Phasen $p_k$ beschreiben. Derartige Modellfunktionen können als sogenannte Jonson-Mehl-Avrami-Gleichungen (beispielsweise aus Buchmayr, Werkstoff- und Produktionstechnik mit Mathcad, ISBN 3-540-43014-8 Springer-Verlag 2002, Kap. 3.4.2, S.87) angesetzt werden; auch aus Wolff/Boettcher/Boehm, Phase transformations in steel in the multiphase case - general modelling and parameter identification, Report 07-02, Universität Bremen (https://www.researchgate.net/publication/237220674_Phase_tra nsformation_in_steel_in_the_multiphase_case_-_general_modelling_and_parameter_identification) sind entsprechende Gleichungssysteme für Modelle zu Phasenumwandlungen in Stahl bekannt: derartige Gleichungssysteme enthalten ihrerseits metallurgische Modellparameter wie z.B. einen zeitlichen Exponenten oder eine Keimbildungsrate, deren Werte innerhalb plausibler Grenzen angenommen werden müssen.

Als Randbedingung für die Wärmeleitungsgleichung (1) wird in der Regel eine Wärmestromdichte $j_o$ für die Oberfläche des Walzguts vorgegeben, auf dessen Volumen sich die Gleichungen (1) bis (4) beziehen: diese Wärmestromdichte $j_o$ beschreibt den Wärmestrom, der von dem Walzgut über dessen Oberfläche mit der Umgebung des Walzguts (z.B. umgebende Luft, auf das Walzgut aufgebrachtes Kühlmittel oder das Walzgut kontaktierende Rollen oder Walzen) ausgetauscht wird; die Wärmestromdichte $j_o$ kann beispielsweise modelliert werden als

$$j_o(T_o) = \varepsilon_s(T_o^4 - T_u^4) + \alpha v^{0,4}(T_o - T_l) + \beta(T_o - T_r) \qquad (5)$$

In Gleichung (5) beschreiben der erste, zweite und dritte Term einen Strahlungsaustausch mit der Umgebung, einen konvektiven Wärmeaustausch mit der Umgebungsluft bzw. einen Wärmeleitungsprozess mit einer das Walzgut kontaktierenden Walze bzw. Rolle. Dabei entspricht $T_o$ der Oberflächentemperatur des Walzguts, $T_u$ der für den Strahlungsaustausch maßgeblichen Umgebungstemperatur, $\alpha$ einem Wärmeübergangskoeffizienten zwischen Walzgut und Umgebungsluft, $T_l$ der Temperatur der Umgebungsluft, $v$ einer Transportgeschwindigkeit des Walzguts relativ zur Umgebungsluft, $\beta$ einem Wärmeleitungskoeffizienten an eine Walze bzw. Rolle und $T_r$ die Temperatur der betreffenden Walze bzw. Rolle. Wiederum müssen für die Größen $\varepsilon_s$, $\alpha$, $\beta$ sowie gegebenenfalls für $T_u$, $T_l$ und $T_r$ im Rahmen der Modellierung des Walzprozesses plausible Werte angenommen werden; diese Größen stellen somit weitere Modellparameter des physikalischen Produktionsmodells, die jedoch nicht zu den vorgenannten metallurgischen Modellparametern y zählen.

Durch gemeinsames Lösen der durch (1) bis (4) vorgegebenen Gleichungen kann - ausgehend von einer angenommenen Anfangswertverteilung für die Temperatur bzw. Enthalpie $T(h^0,p_1^0,...,p_m^0)$ und Anfangswertverteilung der einzelnen Phasenanteile $p_k^0$ sowie unter Beachtung entsprechender Randbedingungen für die Wärmeleitungsgleichung (1) wie z.B. Gleichung (5) - die nach einer bestimmten Zeitspanne resultierende Temperatur- bzw. Enthalpieverteilung $T(h,p_1,...,p_m)$ ermittelt werden.

Die Anfangswertverteilungen können sich beispielsweise auf einen Zeitpunkt kurz vor Eintritt des betrachteten Walzguts in das Walzwerk beziehen, an dem das Walzgut in der Regel eine hohe und gleichmäßig verteilte Temperatur besitzt und ggf. aus nur wenigen metallurgischen Phasen - beispielsweise aus nur einer einzigen austenitischen Phase - besteht. Die Zeitspanne, für die die Gleichungen (1) bis (4) gelöst werden, kann sich beispielsweise auf das Zeitintervall erstrecken, das der Produktionsdauer des Walzguts in dem Walzwerk bis zum Aufhaspeln entspricht, wodurch der gesamte Produktionsprozess in dem Walzwerk rechnerisch modelliert wird.

Alternativ dazu können die Gleichungen (1) bis (4) jeweils für mehrere, unmittelbar aufeinander folgende Zeitintervalle gelöst werden, wobei sich die Gleichungen (1) bis (4) in jedem Zeitintervall unterscheiden. Dabei stellen die Resultate am Ende einer Zeitspanne die Anfangswerte für die unmittelbar darauf folgende Zeitspanne dar. Dadurch wird in jedem Zeitintervall jeweils nur einen Teil des Produktionsprozesses in dem Walzwerk abgebildet. Durch diese Vorgehensweise kann die rechnerische Modellierung des Produktionsprozesses durch entsprechende Auswahl beispielsweise der Wärmeleitungsgleichung oder der Phasenumwandlungsgleichungen besonders gut an den jeweiligen Produktionsabschnitt in dem Walzwerk angepasst werden.

Weiterhin ist zur Ermittlung von mechanischen Eigenschaften eines Walzguts beispielsweise aus Buchmayr, Werkstoff- und Produktionstechnik mit Mathcad, ISBN 3-540-43014-8 Springer-Verlag 2002, Kap. 3.9.2.1 - 3.9.2.4, S.122-123 bekannt, wie sich die Streckgrenze $T_s$ des Walzguts in Abhängigkeit von Fremdelementanteilen im Walzgut bzw. unter den Mechanismen der Mischkristallverfestigung, der Kaltverfestigung, der Feinkornhärtung auf Basis der Hall-Petch-Beziehung oder der Teilchenhärtung ändert. Diese Mechanismen werden dabei in Form von empirischen metallurgischen Gleichungen beschrieben, in denen bestimmte Größen jeweils innerhalb eines bestimmten Wertebereiches variieren werden können. Diese Größen umfassen beispielsweise eine Proportionalitätskonstante bei der Mischkristallverfestigung, eine weitere Proportionalitätskonstante oder einen Korndurchmesser bei der Feinkornhärtung, einen Verfestigungsexponenten bei der Kaltverfestigung oder eine Teilchengröße bei der Teilchenhärtung und stellen ebenfalls metallurgische Modellparameter des physikalischen Produktionsmodells dar. Aus Ibabe, Thin Slab Direct Rolling of Microalloyed Steels, ISBN 0-87849-485-5, Volume 33 of Materials Science Foundations (ISSN 1422-3597), ist beispielsweise bekannt, mittels einer empirischen Gleichung (Eq.44 S.70) aus einer gemittelten Kühlrate eine Ferritkorngröße zu bestimmen.

Weiterhin kann die Zugfestigkeit $Y_s$ oder die Streckgrenze $T_s$ einer bestimmten Phase h eines Walzguts beispielsweise jeweils als Summe aus einem Grundbeitrag $Y_{s,h,0}$ bzw. $T_{s,h,0}$ der Phase h und den Beiträgen $f_{Y,h,i}(c_i)$ bzw. $f_{T,h,i}(c_i)$ einzelner Fremdelementanteile $c_i$ in der betreffenden Phase h ausgedrückt werden:

$$Y_{s,h} = Y_{s,h,0} + \sum_i f_{Y,h,i}(c_i) \tag{6}$$

bzw.

$$T_{s,h} = T_{s,h,0} + \sum_i f_{T,h,i}(c_i) \tag{7}$$

Dabei bezeichnen die Beiträge $f_{Y,h,i}(c_i)$ bzw. $f_{T,h,i}(c_i)$ jeweils monoton steigende Funktionen, die von dem Fremdelementanteil $c_i$ in der Phase h abhängen. Beispielsweise lässt sich der Beitrag von Perlit zur Zugfestigkeit eines Walzguts $Y_{S,P}$ in einen Grundbeitrag aufgrund des Lamellenabstandes innerhalb des Perlits und in einen linear vom Kohlenstoffgehalt $c_P$ im Perlit abhängigen Anteil ausdrücken als

$$Y_{S,P} = \frac{\beta}{\sqrt{S_0}} + \alpha_C \cdot c_P \tag{8}$$

Hierbei bezeichnen $\alpha_C$ und $\beta$ jeweils einen für Perlit spezifischen metallurgischen Modellparameter, der im Rahmen eines bestimmten Modells zu wählen bzw. anzupassen ist, während $S_0$ eine bekannte morphologische Kenngröße von Perlit darstellt.

Die Zugfestigkeit eines Werkstoffs, der aus einer Mischung mehrerer unterschiedlicher Phasen h besteht, lässt sich beispielsweise anhand der Formel

$$Y_{S,total} = \sqrt[n]{\frac{\sum_h (x_h)^n \cdot Y_{S,h}}{\sum_h (x_h)^n}} \qquad\qquad (9)$$

ermitteln, wobei n wiederum ein innerhalb eines bestimmten Modells zu wählender bzw. anzupassender metallurgischer Modellparameter ist. Die Gleichungen (6) bis (9) stellen somit Beispiele für die vorgenannten empirischen metallurgischen Gleichungen dar.

Durch eine Modellierung kann insbesondere der zeitliche Ablauf des Produktionsprozesses bzw. die zeitliche Entwicklung von Eigenschaften des ersten Walzguts während dessen Produktion dargestellt werden. Insbesondere ist es möglich, die zeitliche Entwicklung von Eigenschaften einzelner Abschnitte des ersten Walzgutes darzustellen, sodass der Einfluss wechselnder Produktionsbedingungen auf die mechanischen Eigenschaften des Walzgutes berücksichtigt werden kann.

Beispielsweise können wechselnde Geschwindigkeiten des ersten Walzguts beim Durchlauf durch das Walzwerk, zeitlich nichtkonstante Kühlraten von Kühleinrichtungen oder ein nichtkonstanter Temperaturverlauf des ersten Walzguts in Längsrichtung beim Eintritt in das Walzwerk berücksichtigt werden. Dadurch kann die Einhaltung von zugesicherten Eigenschaften des produzierten ersten Walzguts über seine gesamte Länge besser sichergestellt werden.

Die Daten des weiteren Produktionsdatensatzes $F_f$ umfassen ausschließlich modellierte Daten und Eigenschaften des ersten Walzguts bzw. von Walzgutabschnitten des ersten Walzguts, die messtechnisch nur schlecht oder überhaupt nicht zugänglich sind. Darunter fallen beispielsweise Temperaturen des betreffenden Walzguts bzw. von Walzgutabschnitten an verschiedenen Walzwerksabschnitten während des Produktionsvorganges. Alternativ oder zusätzlich kann der weitere Produktionsdatensatz $F_f$ eine oder mehrere Kühlraten des Walzguts bzw. von Walzgutabschnitten während des Produktionsvorganges umfassen.

Gleichermaßen können auch die metallurgischen Eigenschaften des metallurgischen Datensatzes $K_f$ während des Produktionsprozesses nicht direkt messtechnisch erfasst sondern nur modelliert werden. Dazu zählen z.B. Phasenanteile $p_k$, deren zeitliche Entwicklung beispielsweise anhand des vorgenannten Gleichungssystems (4) ermittelt werden kann.

Weiterhin sind mit Hilfe des physikalischen Produktionsmodells solche Produktionsdaten $P_f$, $P_i$ modellierbar, die zwar prinzipiell vorgegeben oder messtechnisch erfasst werden, die jedoch aufgrund eines Fehlers nicht vorliegen (z.B. unzuverlässig erfassbarer Temperaturwert aufgrund eines Kühlwasserfilms auf einem Walzgut) - in diesem Fall kann der fehlende Wert in einem Produktionsdatensatz durch eine Modellierung anhand des physikalischen Produktionsmodells ersetzt werden.

Mit Hilfe des statistischen Datenmodells ist aus dem Produktionsdatensatz $P_f$ des ersten Walzguts ein zweiter mechanischer Datensatz $S_f$ ermittelbar, der die mechanischen Eigenschaften des ersten Walzguts nach dessen Produktion in dem Walzwerk umfasst. Es handelt sich dabei um dieselbe Gruppe an mechanischen Eigenschaften wie bei den anhand des physikalischen Produktionsmodells ermittelten.

Bei dem statistischen Datenmodell des erfindungsgemäßen Verfahrens handelt es sich um ein trainiertes und validiertes statistisches Datenmodell im vorab definierten Sinn, das in der Lage ist, die mechanischen Eigenschaften des ersten Walzguts nach dessen Produktion in dem Walzwerk in Form eines zweiten mechanischen Datensatzes $S_f$ anhand von erlernten Korrelationen zu ermitteln. Der Ausdruck 'nach der Produktion' ist wieder im vorab definierten Sinne zu verstehen. Der zweite mechanische Datensatz $S_f$ umfasst die gleiche Anzahl und die gleiche Art an mechanischen Eigenschaften wie der erste mechanische Datensatz $C_f$. Die Daten des ersten und zweiten mechanischen Datensatzes $S_f$, $C_f$ entsprechen daher einander und unterscheiden sich voneinander lediglich in der Art, wie sie jeweils ermittelt werden.

Die genannten Korrelationen des statistischen Datenmodells werden diesem vor dessen Verwendung in dem erfindungsgemäßen Verfahren anhand von verfügbaren Produktionsdatensätzen $P_i$ der weiteren Walzgüter und den zugehörigen gemessenen (d.h. in einer Beprobung ermittelten) mechanischen Eigenschaften $B_i$ eingelernt. Anders ausgedrückt bilden einige der Produktionsdatensätzen $P_i$ der weiteren Walzgüter die Trainingsdatensätze des in dem erfindungsgemäßen Verfahren verwendeten statistischen Datenmodells.

In einem ersten Schritt S1 des erfindungsgemäßen Verfahrens werden aus dem Produktionsdatensatz $P_f$ des ersten Walzguts mittels des physikalischen Produktionsmodells der erste mechanische Datensatz $C_f$, der weitere Produktionsdatensatz $F_f$ und der metallurgische Datensatz $K_f$ des ersten Walzguts ermittelt. Die ermittelten Werte des mechanische Datensatzes $C_f$, des weiteren Produktionsdatensatzes $F_f$ und des metallurgischen Datensatzes $K_f$ hängen dabei einerseits von den für die Modellierung des Produktionsprozesses gewählten Gleichungen bzw. Gleichungssystemen und andererseits von den metallurgischen Modellparametern y ab.

In einem zweiten Schritt S2 des erfindungsgemäßen Verfahrens wird aus dem Produktionsdatensatz $P_f$, dem weiteren Produktionsdatensatz $F_f$ und dem metallurgischen Datensatz $K_f$ des ersten Walzguts mittels des trainierten und validierten statistischen Datenmodells der zweite mechanische Datensatz $S_f$ des ersten Walzguts ermittelt.

Der Produktionsdatensatz $P_f$, der weitere Produktionsdatensatz $F_f$ und der metallurgische Datensatz $K_f$ bilden die Eingangsgrößen des statistischen Datenmodells, wohingegen der zweite mechanische Datensatz $S_f$ die Ausgangsgrößen darstellt. Da der weitere Produktionsdatensatz $F_f$ und der metallurgische Datensatz $K_f$ jeweils messtechnisch nicht erfassbare Daten enthalten, bilden sie gemeinsam mit dem Produktionsdatensatz $P_f$ eine erweiterte Datenbasis für das statistische Datenmodell, die eine genauere Vorhersage der mechanischen Eigenschaften durch das statistische Datenmodell ermöglicht, als dies bei einer Verwendung von lediglich des Produktionsdatensatzes $P_f$ als Eingangsgrößen des statistischen Datenmodells möglich wäre.

In einem dritten Schritt S3 des erfindungsgemäßen Verfahrens wird für den Produktionsdatensatz $P_f$ des ersten Walzguts ein gemittelter normierter Abstandswert $d_{f,m}$ zu einer Anzahl n an Produktionsdatensätzen $P_i$ der weiteren Walzgüter bestimmt.

In einem vierten Schritt S4 des erfindungsgemäßen Verfahrens werden mit Hilfe des gemittelten normierten Abstandswertes $d_{f,m}$ die mechanischen Eigenschaften $J_f$ des ersten Walzguts als gewichtetes Mittel aus dem ersten und zweiten mechanischen Datensatz $C_f$, $S_f$ des ersten Walzguts ermittelt. Durch die Gewichtung wird vorteilhaft die Lage der Daten des Produktionsdatensatzes $P_f$ des ersten Walzguts in Relation zu den Daten der Produktionsdatensätze $P_i$ der weiteren Walzgüter berücksichtigt.

Wenn die Daten des Produktionsdatensatzes $P_f$ des ersten Walzguts im Mittel 'in der Nähe' der Daten der Produktionsdatensätze $P_i$ der weiteren Walzgüter liegen, ist dies gleichbedeutend dazu, dass das erste Walzgut eine ähnliche Zusammensetzung besitzt und/oder einen ähnlichen Produktionsprozess durchlaufen hat wie die zur Berechnung des gemittelten normierten Abstandswertes $d_{f,m}$ berücksichtigten weiteren Walzprodukte. In der Folge nimmt der gemittelte normierte Abstandswert $d_{f,m}$ einen kleineren numerischen Wert an im Vergleich zu dem Fall, in dem die Zusammensetzung des ersten Walzguts unähnlicher ist bzw. das erste Walzgut einen stärker abweichenden Produktionsprozess durchlaufen hat als die weiteren Walzprodukte. Zusammengefasst ist das erste Walzgut im ersten Fall in seinen Eigenschaften bzw. seinem Produktionsprozess den weiteren Walzprodukten ähnlicher als im zweiten Fall. Aus diesem Grund ist die Vorhersagegenauigkeit des statistischen Datenmodells im ersten Fall (kleinerer Wert von $d_{f,m}$) höher als im zweiten Fall (größerer Wert von $d_{f,m}$).

Diesem Umstand wird dadurch Rechnung getragen, dass im vierten Schritt S4 des erfindungsgemäßen Verfahrens die von dem statistischen Datenmodell vorhergesagten mechanischen Eigenschaften $S_f$ stärker gewichtet werden als die vom physikalischen Produktionsmodell vorhergesagten mechanischen Eigenschaften $C_f$. In dem Fall, in dem das erste Walzgut den weiteren Walzgütern unähnlicher ist, verschiebt sich dementsprechend die Gewichtung in Richtung der vom physikalischen Produktionsmodell vorhergesagten Werte $C_f$. Die Quotienten aus den einander entsprechenden Werten des zweiten und ersten mechanischen Datensatzes $S_f$ und $C_f$ werden somit proportional zum gemittelten normierten Abstandswert $d_{f,m}$ des ersten Walzguts gewichtet. Mathematisch kann dieser Zusammenhang als $(S_{f,j}/C_{f,j}) \propto d_{f,m}$ ausgedrückt werden, wobei der Index j die jeweils gleiche mechanische Eigenschaft im ersten und zweiten Datensatz (z.B. die Zugfestigkeit $Y_s$) $S_f$ und $C_f$ bezeichnet.

Beispielsweise kann die Ermittlung einer einzelnen mechanischen Eigenschaft $J_{f,j}$ des ersten Walzguts mit Hilfe einer Gewichtungsfunktion $w(d_{f,m})$ gemäß

$$J_{f,j} \;=\; w(d_{f,m}) \cdot S_{f,j} \;+\; (1-w(d_{f,m})) \cdot C_{f,j} \tag{10}$$

ausgedrückt werden, wobei der Index j die betreffende mechanische Eigenschaft in der Gruppe der mechanischen Eigenschaften $J_f$ bezeichnet. Die Gewichtungsfunktion $w_j(d_{f,m})$ ist vom gemittelten normierten Abstandswert $d_{f,m}$ abhängig und kann für jede einzelne mechanische Eigenschaft mit dem Index j beispielsweise in der Form

$$w_j\big(d_{f,m}\big) = 1 - \frac{1}{1 + e^{\lambda_j(\delta_j - d_{f,m})}} \tag{11}$$

separat parametriert werden. Dabei ist der Parameter $\delta_j$ jenes Funktionsargument, bei dem die Gewichtungsfunktion $w_j(d_{f,m})$ den Wert 0,5 annimmt. Der Parameter $\lambda_j$ beeinflusst die Steigung der Funktion. Die Parameter $\delta_j$ und $\lambda_j$ sind aufgrund der Vorhersagen des statistischen Datenmodells auf Basis der Testdaten so zu wählen, dass bei Überschreiten von jeweiligen vordefinierten Fehlertoleranzen in Bezug auf die jeweilige mechanische Eigenschaften $j_f$ die Gewichtungsfunktion $w_j(d_{f,m})$ entsprechend parametriert ist: beispielsweise soll die Gewichtungsfunktion $w_j(d_{f,m})$ bei einer angenommenen halben Fehlertoleranz von 15MPa für die Zugfestigkeit $Y_s$ den Wert 0,5 und bei der angenommenen vollen Fehlertoleranz von 30MPa den Wert 0,1 annehmen, woraus die entsprechenden Werte für $\delta_j$ und $\lambda_j$ folgen.

Gemäß einer bevorzugten Ausgestaltung des erfindungsgemäßen Verfahrens umfassen die mechanischen Eigenschaften $J_f$ des ersten Walzguts eine Zugfestigkeit $Y_s$ und/oder eine Streckgrenze $T_s$ und/oder eine Bruchdehnung

$A_l$. Diese mechanischen Eigenschaften $J_f$ können in einfacher Weise auch bei einer physischen Beprobung des ersten Walzguts ermittelt werden, wodurch vorteilhaft ein direkter Vergleich mit den durch das hybride Modell vorhergesagten Werten möglich ist.

In einer bevorzugten Ausgestaltung des erfindungsgemäßen Verfahrens wird der gemittelte normierte Abstandswert $d_{f,m}$ - beispielsweise als arithmetisches Mittel - aus n normierten Abstandswerten $d_{f,i}$ des Produktionsdatensatzes $P_f$ des ersten Walzguts zu jeweils einem Produktionsdatensatz $P_i$ eines der weiteren Walzgüter gebildet.

Ein normierter Abstandswert $d_{f,i}$ des Produktionsdatensatzes $P_f$ zum Produktionsdatensatz $P_i$ lässt sich beispielsweise gemäß

$$d_{\mathrm{f,i}} = \frac{1}{M} \sum_{k=1}^{M} |z_{\mathrm{k,f}} - z_{\mathrm{k,i}}| \tag{12}$$

ermitteln, wobei $z_{k,f}$ und $z_{k,i}$ jeweils ein standardisierter Wert mit dem Index k aus dem Produktionsdatensatz $P_f$ bzw. aus dem Produktionsdatensatz $P_i$ ist (beispielsweise einen Fremdelementanteil, eine Geschwindigkeit oder eine Temperatur des betreffenden Walzguts während dessen Produktion) und wobei M die Anzahl der berücksichtigten Daten eines Produktionsdatensatzes bezeichnet. In Formel (12) bezeichnet der Index f zu dem ersten Walzgut gehörende Größen, der Index k bezeichnet eine Komponente im Produktionsdatensatz $P_f$ bzw. $P_i$, während der Index i zu den weiteren Walzgütern gehörende Größen kennzeichnet. Weiterhin lässt sich der standardisierte Wert $z_{k,f}$ in Formel (12) gemäß

$$z_{\mathrm{k,f}} = \frac{x_{k,f} - x_{k,min}}{x_{k,max} - x_{k,min}} \tag{13a}$$

berechnen, wobei $x_{k,min}$ bzw. $x_{k,max}$ den kleinsten bzw. den größten Wert der Komponente mit dem Index k in der Gesamtheit aller Produktionsdatensätze $P_i$ der weiteren Walzgüter bezeichnet. Anders ausgedrückt: die Komponente $x_{k,f}$ mit dem Index k im Produktionsdatensatz $P_f$ des ersten Walzguts wird zu dem Wertebereich in Beziehung gesetzt, der durch den kleinsten und größten Wert derselben Komponente (Index k) aus der Gesamtheit der weiteren Walzgüter aufgespannt wird. Für die standardisierte Eigenschaft $z_{k,i}$ in Formel (12) gilt eine zu Formel (13a) analoge Formel:

$$z_{\mathrm{k,i}} = \frac{x_{k,i} - x_{k,min}}{x_{k,max} - x_{k,min}} \tag{13b}$$

In Formel (13b) bezeichnet $x_{k,i}$ eine Komponente mit Index k im Produktionsdatensatz $P_i$ eines weiteren Walzguts und $z_{k,i}$ einen entsprechenden standardisierten Wert in Produktionsdatensatz des weiteren Walzguts.

Der normierte Abstandswert $d_{f,i}$ stellt somit ein dimensionsloses Maß zwischen dem Produktionsdatensatz $P_f$ des ersten Walzguts und einem Produktionsdatensatz $P_i$ eines der weiteren Walzgüter dar. Unter 'normiert' ist in diesem Zusammenhang zu verstehen, dass der Abstandswert unabhängig von der konkreten Anzahl und physikalischen Einheit der jeweiligen Daten in den einzelnen Produktionsdatensätzen ermittelbar ist. Unter 'dimensionslos' ist zu verstehen, dass die betreffende Größe mit keiner physikalischen Maßeinheit behaftet ist. Im Gegensatz zu einer dimensionslosen Größe sind die einzelnen Daten von Produktionsdatensätzen in der Regel mit physikalischen Einheiten versehen; so besitzt beispielsweise ein Walzspalt die physikalische Einheit einer Länge, eine Kühlrate wird in den physikalischen Einheiten Temperatur pro Zeiteinheit gemessen, eine Kühlmittelrate bemisst sich in Volumen pro Zeit etc. Weiterhin wird unter dem Begriff 'Maß' eine mathematische Berechnungsvorschrift verstanden, die eine Mehrzahl von reellen Größen auf die Menge der positiven reellen Zahlen abbildet.

Aus einer Anzahl n an kleinsten normierten Abstandswerten $d_{f,i}$ des Produktionsdatensatzes $P_f$ des ersten Walzguts zu jeweils einem Produktionsdatensatz $P_i$ eines der weiteren Walzgüter wird anhand des arithmetischen Mittelwertes

$$d_{\mathrm{f,m}} = \frac{1}{n} \sum_{i=1}^{n} d_{\mathrm{f,i}} \tag{14}$$

der gemittelte normierte Abstandswert $d_{f,m}$ gebildet. Gemäß der bevorzugten Ausgestaltung des erfindungsgemäßen Verfahrens umfasst die Anzahl n der dabei berücksichtigten Produktionsdatensätzen $P_i$ der weiteren Walzgüter 0,1 bis 0,5 Prozent der Trainingsdatensätze des trainierten statistischen Datenmodells.

In der Praxis umfasst die Menge der Trainingsdatensätze, die zum Trainieren des statistischen Datenmodells herangezogen wird, mehrere Tausend von dem Walzwerk innerhalb eines zusammenhängenden Zeitraums produzierte Walzgüter, was beispielsweise einem Produktionszeitraum von 1 bis 2 Jahren entspricht. Daraus ergibt sich, dass zur Ermittlung des gemittelten normierten Abstandswertes $d_{f,m}$ etwa 50 - 100 Produktionsdatensätze aus der Menge der Trainingsdatensätze herangezogen werden, mit denen das statistische Datenmodell trainiert worden ist.

Das erste Walzgut und die weiteren Walzgüter können datentechnisch in einen oder mehrere Walzgutabschnitte unterteilt werden. Ein Walzgutabschnitt weist dabei bevorzugt eine Länge von wenigen Metern, beispielsweise von zwei bis fünf Metern auf. Auch eine Unterteilung in längere oder kürzere Walzgutabschnitte ist möglich. Die Unterteilung erfolgt in der Regel in Längsrichtung des Walzguts, die während dessen Produktion mit der Produktionsrichtung im Walzwerk übereinstimmt. Im einfachsten Fall ist ein Walzgut in nur einen einzigen Walzgutabschnitt unterteilt. Die Daten eines jeweiligen Produktionsdatensatzes können dementsprechend pro Walzgutabschnitt erfasst und datentechnisch dem entsprechenden Walzgutabschnitt zugeordnet werden. Gleichermaßen können auch die Daten eines weiteren Produktionsdatensatzes und eines metallurgischen Datensatzes pro Walzgutabschnitt modelliert und datentechnisch dem entsprechenden Walzgutabschnitt zugeordnet werden.

In dem Fall, dass die Daten des Produktionsdatensatzes eines Walzguts, des zugehörigen weiteren Produktionsdatensatzes und des zugehörigen metallurgischen Datensatzes für einen oder mehrere Walzgutabschnitte vorliegen, ist es auch möglich, die daraus ermittelten mechanischen Eigenschaften des betreffenden Walzguts entsprechend dem einen oder mehreren Walzgutabschnitten zu ermitteln. Gemäß einer weiteren bevorzugten Ausgestaltung des erfindungsgemäßen Verfahrens werden daher die mechanischen Eigenschaften $J_f$ des ersten Walzguts für zumindest einen Walzgutabschnitt des ersten Walzguts ermittelt. Dadurch kann eine noch genauere Modellierung des Produktionsprozesses erzielt und die Ermittlung der mechanischen Eigenschaften $J_f$ des ersten Walzguts in Abhängigkeit von der Position eines Walzgutabschnitts innerhalb des Walzguts erreicht werden.

Das Walzwerk weist eine bestimmte Anzahl an Walzgerüsten auf, wobei in Produktionsrichtung gesehen ein erstes Walzgerüst von einem Walzgut als erstes und ein letztes Walzgerüst als letztes durchlaufen wird. Weiterhin weist das Walzwerk in der Regel eine bestimmte Anzahl an Kühlvorrichtungen, eine bestimmte Anzahl an Walzwerksabschnitten und zumindest eine Haspelvorrichtung auf. Ein Walzwerksabschnitt kann sich beispielsweise zwischen einem der Walzgerüste des Walzwerks und dem - in Produktionsrichtung gesehen - unmittelbar nachgeordneten Walzgerüst oder vom letzten Walzgerüst bis zu einer Haspelvorrichtung des Walzwerks erstrecken.

In einer bevorzugten Ausgestaltung des erfindungsgemäßen Verfahrens, bei dem das Walzwerk N Walzgerüste $R_1,...,R_N$, L Kühlvorrichtungen $Q_1,...,Q_L$, Z Walzwerksabschnitte $A_1,...,A_Z$ und zumindest eine Haspelvorrichtung aufweist, umfassen die Produktionsdatensätze des ersten und der weiteren Walzgüter $P_f$, $P_i$ jeweils zumindest eine der folgenden Stell- oder Messgrößen für zumindest einen Walzgutabschnitt des ersten und der weiteren Walzgüter:

- einen oder mehrere Fremdelementanteile, wie beispielsweise Gewichtsprozent an Kohlenstoff, Silizium, Mangan, Kupfer, Nickel, Titan, Chrom, Schwefel, Phosphor, Kalzium, etc., in dem jeweiligen Walzgutabschnitt,
- eine Dicke und/oder eine Breite des Walzgutabschnitts vor dem Eintritt in ein erstes Walzgerüst $R_1$ des Walzwerks,
- eine Dicke und/oder eine Breite des Walzgutabschnitts nach dem Austritt aus einem letzten Walzgerüst $R_N$ des Walzwerks,
- zumindest eine messtechnisch erfasste Temperatur des jeweiligen Walzgutabschnitts, bevorzugt eine unmittelbar vor dem Eintritt in das Walzwerk erfasste und/oder eine in einem der Walzwerksabschnitte $A_1,...,A_Z$ (z.B. anhand einer pyrometrischen Messung) erfasste und/oder eine beim Aufhaspeln des ersten bzw. der weiteren Walzgüter erfasste Temperatur des jeweiligen Walzgutabschnitts,
- zumindest einen Walzspaltwert eines der Walzgerüste $R_1,...,R_N$ beim Durchtritt des Walzgutabschnitts durch das jeweilige Walzgerüst $R_1,...,R_N$ des Walzwerks,
- zumindest einen von einer der Kühlvorrichtungen $Q_1,...,Q_L$ des Walzwerks abgegebenen Kühlmittelstrom beim Durchtritt des Walzgutabschnitts durch den Wirkbereich der jeweiligen Kühlvorrichtung $Q_1,...,Q_L$, oder
- zumindest eine Geschwindigkeit des Walzgutabschnitts in einem der Walzwerksabschnitte $A_1,...,A_Z$ des Walzwerks.

Durch die Berücksichtigung einzelner Gewerke des Walzwerks bzw. einzelner Einflussfaktoren bei der Modellierung des Produktionsprozesses kann eine noch genauere Ermittlung der mechanischen Eigenschaften $J_f$ des ersten Walzguts erreicht werden.

Die genannten Stell- oder Messgrößen werden jeweils während der Produktion eines Walzgutabschnitts des ersten bzw. der weiteren Walzgüter in dem Walzwerk datentechnisch erfasst und dem entsprechenden Walzgutabschnitt datentechnisch zugeordnet. Die genannten Stell- oder Messgrößen sind mess- oder datentechnisch auch bei rauen Produktionsbedingungen in der Regel gut und dauerhaft erfassbar und stellen daher für das statistische Datenmodell verlässlich verfügbare Eingangsdaten dar, bei denen mit nur sehr selten mit Datenfehlern zu rechnen ist.

Unter dem Walzspaltwert eines Walzgerüsts wird der Abstand zwischen zwei Arbeitswalzen eines Walzgerüsts in Dickenrichtung des Walzguts bei dessen Durchtritt durch das betreffende Walzgerüst verstanden. Unter dem Wirkbereich einer Kühleinrichtung wird jener Bereich innerhalb des Walzwerks verstanden, in dem die betreffende Kühlvorrichtung eine Kühlwirkung des durch das Walzwerk durchtretenden Walzguts bzw. des Walzgutabschnitts hervorruft.

Beispielsweise kann der Wirkbereich einer Kühlvorrichtung mit deren Sprühbereich zusammenfallen. Weiterhin kann das Walzgut bei der Produktion in den einzelnen Walzwerksabschnitten $A_1,...,A_Z$ aufgrund der von den jeweiligen Walzgerüsten durchgeführten Dickenreduktion jeweils eine unterschiedliche Geschwindigkeit aufweisen. Die Geschwindigkeiten in den einzelnen Walzwerksabschnitten können entweder auf direkte Weise messtechnisch erfasst werden (z.B. mit Hilfe von berührungslosen Geschwindigkeitsmessverfahren) oder beispielsweise aus einer Umdrehungsgeschwindigkeit und einem Durchmesser der Arbeitswalzen eines Walzgerüsts und der Dickenreduktion des Walzguts an dem betreffenden Walzgerüst ermittelt werden.

Gemäß einer weiteren bevorzugten Ausgestaltung des erfindungsgemäßen Verfahrens umfasst der weitere Produktionsdatensatz $F_f$ für zumindest einen Walzgutabschnitt des ersten Walzguts zumindest eine modellierte Kühlrate $q_z$ und/oder zumindest einen modellierten Temperaturwert $T_z$ des Walzgutabschnitts beim Passieren von zumindest einem der Walzwerksabschnitte $A_1,...,A_Z$ des Walzwerks.

In einer weiteren bevorzugten Ausgestaltung des erfindungsgemäßen Verfahrens umfassen die metallurgischen Eigenschaften des metallurgischen Datensatzes $K_f$ des ersten Walzguts Phasenanteile $p_h$ und/oder morphologische Kenngrößen $a_k$ (wie z.B. einen Lamellenabstand im Perlit, eine Ferrit- oder Austenitkorngröße, eine Plattendicke im Bainit, eine Paketgröße des Martensit, eine gemittelte Versetzungsdichte oder einen Rekristallisationsgrad) verschiedener metallurgischer Phasen des ersten Walzguts bzw. zumindest eines Walzgutabschnitts des ersten Walzguts. Die metallurgischen Phasenanteile $p_k$ werden beispielsweise in Gewichtsprozent angegeben.

Beispielsweise kann die Entwicklung einer morphologischen Kenngröße $a_h$ eines Walzguts beim Produktionsprozess in einem Walzwerk durch eine Gleichung der Form

$$\frac{da_h}{dt} = f_1(T1, \{a_h\}) + f_2(T2, \{a_h\}, \dot{\varphi}) \qquad (15)$$

beschrieben werden, wobei der Index h eine bestimmte Phase, beispielsweise Austenit, Ferrit oder Perlit, repräsentiert. In Gleichung (15) bezeichnet $f_1$ eine mathematische Funktion, die die zeitliche Entwicklung der morphologischen Kenngröße $a_h$ in Abhängigkeit der vorherrschenden morphologischen Kenngrößen (durch das Symbol $\{a_h\}$ dargestellt) und der Temperatur T1 außerhalb eines Walzspalts beschreibt. Demgegenüber bezeichnet $f_2$ eine Funktion, die die Gefügeentwicklung durch Umformung in einem Walzspalt in Abhängigkeit der gegebenen Morphologie $\{a_h\}$, der zeitlichen Ableitung des Umformungsgrads $\partial_t\varphi$ des Walzguts im Walzspalt und einer Temperatur T2 im Walzspalt beschreibt.

Nach der Produktion in dem Walzwerk, insbesondere nach der Produktion in einem Warmwalzwerk, besitzt das produzierte Walzgut in der Regel eine erhöhte Temperatur von bis zu 800°C. Anschließend wird das Walzgut in der Regel gelagert oder aktiv gekühlt, bis es eine Umgebungstemperatur erreicht hat. Erst nachdem dieser Temperaturausgleich abgeschlossen ist, findet in der Regel eine Beprobung statt, anhand der die von einem Modell ermittelten mechanischen Eigenschaften mit den gemessenen mechanischen Eigenschaften eines physischen Probenstücks verglichen werden können. Während der Abkühlphase auf Umgebungstemperatur finden im Material des Walzguts Karbid- und Nitridausscheidungen statt, die die mechanischen Eigenschaften noch nach dem abgeschlossenen Produktionsprozess im Walzwerk beeinflussen. Insbesondere bewirken Karbid- und Nitridausscheidungen mit einer Größe von bis zu 10nm eine Verfestigung des Materials.

Um daher die anhand einer Beprobung ermittelten mechanischen Eigenschaften mit den von einem Modell vorhergesagten mechanischen Eigenschaften $J_f$ eines ersten Walzguts besser vergleichen zu können, werden gemäß einer weiteren bevorzugten Ausführungsform des erfindungsgemäßen Verfahrens in einem fünften Schritt S5 die ermittelten mechanischen Eigenschaften $J_f$ des ersten Walzguts einer Abkühlungskorrektur unterzogen, bei der Karbid- und Nitridausscheidungen in dem ersten Walzgut während des Abkühlens nach der Produktion in dem Walzwerk berücksichtigt werden.

Die Ausscheidungsprozesse sind zeit- und temperaturabhängig. Insbesondere in dem Fall, dass das produzierte Walzgut die Auskühlphase in Form eines zu einem Coil gewickelten Bandes durchläuft, unterscheiden sich die Ausscheidungsmengen und die damit verbundene Ausscheidungshärtung über Bandlänge aufgrund der unterschiedlichen Abkühlbedingungen für unterschiedliche Bandpositionen im Coil. Besonders bevorzugt wird eine Abkühlungskorrektur auf einzelne Walzgutabschnitte eines Walzguts angewandt, da die Walzgutabschnitte zu einer jeweiligen Bandposition des Materials in einem Coil korrespondieren. Dadurch kann das unterschiedliche Abkühlverhalten einzelner Walzgutabschnitte besonders genau abgebildet werden.

Die Abkühlungskorrektur wird beispielsweise mittels tabellierter Korrekturfunktionen durchgeführt, die anhand eines weiteren metallurgischen Modells vorab, d.h. unabhängig vom tatsächlichen Produktionsprozess eines Walzguts, ermittelt werden. Jede der mechanischen Eigenschaften $J_f$ wird mit einer eigenen Korrekturfunktion korrigiert. Der Funktionswert einer Korrekturfunktion stellt beispielsweise einen Multiplikationsfaktor dar, mit dem jede der unkorrigierten mechanischen Eigenschaften $J_f$ beaufschlagt werden muss.

Die Argumente der Korrekturfunktionen (also deren Eingangsgrößen) sind beispielsweise bestimmte Legierungsbestandteile (insbesondere Niob, Titan und Vanadium) eines Walzguts, eine Haspeltemperatur des Walzguts bzw.

eines Walzgutabschnitts sowie der Innen- und Außenradius des betreffenden Coils. 'Tabelliert' bedeutet in diesem Zusammenhang, dass die Korrekturfunktionen nur für eine bestimmte Anzahl von Werten (z.B. für 10-50 Werte) eines Arguments ermittelt wird. Bei zwischen diesen Werten liegenden Eingangsgrößen werden die Korrekturen für jeder der mechanischen Eigenschaften $J_f$ des ersten Walzguts durch entsprechende Interpolation der Funktionswerte ermittelt.

Der Bereich der Haspeltemperatur, für den die Korrekturfunktionen ermittelt werden, liegt bevorzugt in einem Bereich von 600°C bis 750°C, wobei unterhalb von 600°C die für die Korrekturfunktionen maßgebliche Ausscheidungskinetik vernachlässigbar ist und 750°C einer maximalen Haspeltemperatur in der Praxis entsprechen. Weiterhin wird bei der Ermittlung der Korrekturfunktionen von einer vollständigen Phasenumwandlung des Walzgutmaterials vor dem Haspeln ausgegangen, d.h. die Effekte einer Perlit- bzw. Zementitbildung im Walzgut werden nicht berücksichtigt.

In dem erfindungsgemäßen Verfahren zur Erstellung eines hybriden Modells korrespondiert das hybride Modell im Wesentlichen mit dem hybriden Modell aus dem vorgenannten erfindungsgemäßen Verfahren zur Bestimmung mechanischer Eigenschaften $J_f$ eines ersten Walzgutes.

Bei dem erfindungsgemäßen Verfahren zur Erstellung eines hybriden Modells für die Bestimmung mechanischer Eigenschaften $J_f$ eines in einem Walzwerk, insbesondere in einem Warmwalzwerk, produzierten ersten Walzguts umfasst das hybride Modell dementsprechend Produktionsdatensätze $P_i$ weiterer in dem Walzwerk produzierter Walzgüter, ein physikalisches Produktionsmodell und ein statistisches Datenmodell.

Die Produktionsdatensätze $P_i$ der weiteren Walzgüter werden in dem vorab definiertem Sinn verstanden, wobei die weiteren Walzgüter wiederum eine ersten Menge $I_1$ bilden. Weiterhin liegt zu jedem der Produktionsdatensätze $P_i$ der weiteren Walzgüter ein korrespondierender Beprobungsdatensatz $B_i$ des betreffenden Walzguts vor, der jeweils die nach der Produktion des weiteren Walzguts in dem Walzwerk mittels einer Beprobungsvorrichtung ermittelten mechanischen Eigenschaften des weiteren Walzguts umfasst.

Das physikalische Produktionsmodell weist für die Modellierung des Produktionsprozesses der weiteren Walzgüter einen Satz von metallurgische Modellparametern y in oben definiertem Sinn auf, sodass damit aus den Produktionsdatensätzen $P_i$ jeweils ein erster mechanischer Datensatz $C_i$, umfassend die jeweiligen mechanischen Eigenschaften nach der Produktion in dem Walzwerk, ein weiterer Produktionsdatensatz $F_i$, umfassend weitere Produktionsdaten während der Produktion in dem Walzwerk, und ein metallurgischer Datensatz $K_i$, umfassend metallurgische Eigenschaften während der Produktion in dem Walzwerk, für jedes der weiteren Walzgüter ermittelbar sind. Die Begriffe 'während der Produktion' und 'nach der Produktion' sind wieder im vorab beschriebenen Sinn zu verstehen.

Weiterhin umfasst das erfindungsgemäße Verfahren zur Erstellung eines hybriden Modells ein statistisches Datenmodell, mit dessen Hilfe zu jedem der weiteren Walzgüter aus dem jeweiligen Produktionsdatensatz $P_i$ des weiteren Walzguts ein zweiter mechanischer Datensatz $S_i$ ermittelbar ist, der die mechanischen Eigenschaften des weiteren Walzguts nach dessen Produktion in dem Walzwerk umfasst. Bei dem statistischen Datenmodell handelt es sich zu Beginn des erfindungsgemäßen Verfahrens um ein noch untrainiertes und nicht validiertes statistisches Datenmodell im vorab definierten Sinn.

In einem ersten Schritt S1' des erfindungsgemäßen Verfahrens zur Erstellung eines hybriden Modells werden in einer oder mehreren aufeinanderfolgenden Iterationen mit Hilfe des physikalischen Produktionsmodells zu jedem der Produktionsdatensätze $P_i$ der weiteren Walzgüter der jeweils korrespondierende erste mechanische, weitere Produktions- und metallurgische Datensätze $C_i$, $F_i$, $K_i$ ermittelt, d.h. in vorgenanntem Sinn modelliert. Der Begriff 'Iteration' ist dabei algorithmisch als das Durchführen derselben wiederkehrenden Berechnungsvorgänge - im konkreten Fall das Ermitteln der genannten Datensätze - zu verstehen.

Wiederum hängen dabei - wie voranstehend zum erfindungsgemäßen Verfahren zur Bestimmung mechanischer Eigenschaften $J_f$ eines ersten Walzguts ausgeführt - die Daten des jeweiligen ersten mechanischen Datensatzes $C_i$, des jeweiligen weiteren Datensatzes $F_i$ und des jeweiligen metallurgischen Datensatzes $K_i$ in mathematischer Hinsicht explizit von den metallurgischen Modellparametern y ab, wobei diese Abhängigkeit durch die Notation $C_i(\gamma)$, $F_i(\gamma)$ bzw. $K_i(\gamma)$ ausgedrückt werden kann.

Nach jeder Iteration wird für die Gesamtheit der in der zuletzt durchgeführten Iteration ermittelten ersten mechanischen Datensätze $C_i$ der weiteren Walzgüter mittels einer ersten Maßfunktion $\varepsilon_1$ komponentenweise ein (in vorgehend definiertem Sinn mathematisches) Maß für die Abweichung von den korrespondierenden Beprobungsdatensätzen $B_i$ der weiteren Walzgüter ermittelt. 'Komponentenweise' bedeutet in diesem Zusammenhang, dass das Maß für die Abweichung für jede einzelne mechanische Eigenschaft in den ersten mechanischen Datensätzen $C_i$ bzw. in den Beprobungsdatensätzen $B_i$ separat ermittelt wird. Die erste Maßfunktion $\varepsilon_1$ kann dabei als quadratische Form ausgestaltet sein, beispielsweise als

$$\varepsilon_1 = \frac{\sqrt{\Sigma_i\left(C_{i,j}(\gamma)-B_{i,j}\right)^2 \cdot w_i}}{\sqrt{\Sigma_i\left(C_{i,j}(\gamma)\right)^2 \cdot w_i}} \qquad (16)$$

oder

$$\varepsilon_1 = \frac{\sqrt{\sum_i \left(C_{i,j}(\gamma) - B_{i,j}\right)^2 \cdot w_i}}{\sqrt{\sum_i \left(C_{i,j}(\gamma) + B_{i,j}\right)^2 \cdot w_i}} \qquad\qquad (17)$$

wobei der Index i ein konkretes Walzgut der weiteren Walzgüter bezeichnet und der Ausdruck $w_i$ ein individueller Gewichtungsfaktor für den ersten mechanischen Datensatz $C_i$ und den Beprobungsdatensatz $B_i$ des weiteren Walzguts mit Index i ist. Im einfachsten Fall können alle Gewichtungsfaktoren $w_i$ gleich 1 gesetzt werden.

In dem Fall, in dem der Wert der ersten Maßfunktion $\varepsilon_1$ für zumindest eine einzelne mechanische Eigenschaft (in Formeln (16) und (17) jeweils durch den Index j gekennzeichnet) in den ersten mechanischen Datensätzen $C_i$ der weiteren Walzgüter größer als eine vorgegebene Schranke $\varepsilon_{max}$ ist, werden die metallurgischen Modellparameter y variiert und in einer weiteren Iteration werden die ersten mechanischen, die weiteren Produktions- und die metallurgischen Datensätze $C_i$, $F_i$, $K_i$ der weiteren Walzgüter anhand des physikalischen Produktionsmodells mit den geänderten metallurgischen Modellparametern y erneut ermittelt. Die vorgegebene Schranke $\varepsilon_{max}$ kann beispielsweise als ein Zwei- bis Dreifaches der Messgenauigkeit einer der bei einem Beprobungsvorgang ermittelten mechanischen Eigenschaften $B_{i,j}$ betragen. Beispielsweise kann die vorgegebene Schranke $\varepsilon_{max}$ zwei bis drei Megapascal betragen.

Der Ausdruck 'variiert' bedeutet in diesem Zusammenhang, dass die Zahlenwerte der metallurgischen Modellparameter y geändert werden. Durch das Variieren soll der Produktionsprozess der weiteren Walzgüter in der folgenden Iteration genauer modelliert und der Wert der ersten Maßfunktion $\varepsilon_1$, die als Maß für die Genauigkeit der Modellierung herangezogen wird, minimiert werden. Eine entsprechende Minimierung des Wertes der ersten Maßfunktion $\varepsilon_1$ entspricht eine sogenannten 'least squares fit'-Verfahren und kann beispielsweise durch ein Gradientenverfahren nach Levenberg-Marquart (entsprechend http://people.duke.edu/~hpgavin/ce281/lm.pdf) erreicht werden.

In dem Fall, in dem der Wert der ersten Maßfunktion $\varepsilon_1$ für alle mechanischen Eigenschaften in den ersten mechanischen Datensätzen $C_i$ der weiteren Walzgüter kleiner oder gleich der Schranke $\varepsilon_{max}$ ist, ist eine ausreichend genaue Simulation des Produktionsprozesses für die Gesamtheit der weiteren Walzgüter gegeben. Daher werden die Produktionsdatensätze $P_i$ der weiteren Walzgüter zusammen mit den in der zuletzt durchgeführten Iteration ermittelten weiteren Produktions- und metallurgischen Datensätze $F_i$ und $K_i$ der weiteren Walzgüter dem statistischen Datenmodell als Eingangsgrößen und die dazu korrespondierenden Beprobungsdatensätze $B_i$ als Zielgrößen übermittelt. Die Begriffe 'Eingangsgrößen' und 'Zielgrößen' sind wieder im vorgenannten Sinne in Bezug auf statistische Datenmodelle zu verstehen. Dabei entspricht die Anzahl der unterschiedlichen Zielgrößen, die dem statistischen Datenmodell übermittelt werden, der Anzahl der in jedem Beprobungsdatensatz vorkommenden mechanischen Eigenschaften: beispielsweise werden dem statistischen Datenmodell in dem Fall, dass die Beprobungsdatensätze $B_i$ jeweils eine Zugfestigkeit $Y_s$, eine Streckgrenze $T_s$ und eine Bruchdehnung $A_l$ umfassen, drei Zielgrößen übermittelt.

Das statistische Datenmodell umfasst dementsprechend pro Zielgröße ein eigenes statistisches Teildatenmodell, das unabhängig von den anderen Teildatenmodellen existiert und nach entsprechendem Training die jeweils entsprechende mechanische Größe vorherzusagen imstande ist. Die Eingangsgrößen sind im Rahmen des Trainierens der Teildatenmodelle jedoch für alle Teildatenmodelle dieselben.

In einem zweiten Schritt S2' wird das statistische Datenmodell anhand der übermittelten Eingangs- und Zielgrößen mit Hilfe eines ersten maschinellen Lernverfahrens $V_1$ trainiert. Dabei kann jedes Teildatenmodell anhand desselben ersten maschinellen Lernverfahrens $V_1$ trainiert werden, die maschinellen Lernverfahren können alternativ auch pro Teildatenmodell unterschiedlich sein. Die Bezeichnung 'erstes' maschinelles Lernverfahren ist in diesem Zusammenhang als jenes Lernverfahren zu verstehen, das zum Zwecke des Trainierens des Datenmodells bzw. eines Teildatenmodells verwendet wird.

Aus der Fachliteratur sind verschiedene maschinelle Lernverfahren, wie z.B. Verfahren auf Grundlage von Entscheidungsbäumen wie Random Forest oder Gradient Boosting Trees, sogenannte Support Vector Machines, künstliche neuronale Netze, evolutionäre Algorithmen oder auch Kombinationen mehrerer Datenmodelle (Ensemble Learning) bekannt. Beispielhaft sei als Offenlegung für den Vorgang des maschinellen Lernens Friedman et al., The elements of statistical learning, Springer series in statistics, 2001, ISBN 0-387-95284-5, angeführt.

In einem dritten Schritt S3' wird das trainierte statistische Datenmodell anhand von Produktionsdatensätzen $P_i$ und Beprobungsdatensätzen $B_i$ von Walzgütern aus einer zweiten, zur ersten Menge $I_1$ disjunkten zweiten Menge $I_2$ validiert. Dazu werden zu den Walzgütern der zweiten Menge $I_2$ mittels des physikalischen Produktionsmodells die weiteren Produktionsdatensätze $F_i$ und metallurgischen Datensätze $K_i$ ermittelt, die zusammen mit den Produktionsdatensätzen $P_i$ der Walzgüter der zweiten Menge $I_2$ dem trainierten statistischen Datenmodell als Eingangsgrößen zugeführt werden. Das trainierte statistische Datenmodell ermittelt daraus korrespondierende zweite mechanische Datensätze $S_i$ zu den Walzgütern der zweiten Menge $I_2$. Für diese zweiten mechanischen Datensätze $S_i$ wird anhand einer zweiten Maßfunktion $\varepsilon_2$ komponentenweise ein Maß für eine Abweichung zu den dazu korrespondierenden Beprobungsdaten-

sätzen $B_i$ ermittelt. Der Ausdruck 'komponentenweise' ist in der vorab beschriebenen Bedeutung zu verstehen, d.h. es wird für jede einzelne mechanische Eigenschaft in den zweiten mechanischen Datensätzen $S_i$ mittels der zweiten Maßfunktion $\varepsilon_2$ eine Abweichung zur jeweils entsprechenden mechanischen Eigenschaft in den korrespondierenden Beprobungsdatensätzen $B_i$ ermittelt.

Beispielsweise wird für die zweite Maßfunktion $\varepsilon_2$ der mittlere absolute Fehler MAE entsprechend

$$\varepsilon_2 = MAE = \frac{1}{n}\sum_i |S_{i,j} - B_{i,j}| \qquad (18)$$

verwendet, wobei der Index i die Datensätze der Walzgüter aus der zweiten Menge $I_2$ und der Index j eine einzelne mechanische Eigenschaft (z.B. eine Zugfestigkeit $Y_s$, eine Streckgrenze $T_s$ oder eine Bruchdehnung $A_l$) innerhalb der einzelnen Datensätze $S_i$ bzw. $B_i$ bezeichnet.

Die Produktionsdatensätze $P_i$ und Beprobungsdatensätze $B_i$ zweiten Menge $I_2$ stellen somit den Testdatensatz für das statistische Datenmodell dar. Für die Durchführung des zweiten und dritten Schrittes S2' und S3' wird beispielsweise die sich über einen Zeitraum von insgesamt 2 Jahren erstreckende Datengrundlage eines Walzwerks in zwei Mengen aufgeteilt, wobei die Aufteilung dabei innerhalb zusammenhängender Produktionszeiträume erfolgt: beispielsweise können die zur Verfügung stehenden Produktionsdatensätze $P_i$ als Eingangsgrößen und Beprobungsdatensätze $B_i$, als die die Trainingsdatensätze für das statistische Datenmodell bilden, sich über die ersten 1,5 Jahre des Zeitraums erstrecken, während der Testdatensatz die Produktionsdatensätze $P_i$ und Beprobungsdatensätze $B_i$ von Walzgütern der letzten 6 Monate enthält.

Das erste maschinelle Lernverfahren $V_1$, das dem statistischen Datenmodell (bzw. einem jeweiligen Teildatenmodell) zugrunde gelegt wird, kann von vornherein fest gewählt werden. Alternativ ist es auch möglich, das statistische Datenmodell (bzw. jedes Teildatenmodell) auf Grundlage desselben Trainingsdatensatzes mittels verschiedener maschineller Lernverfahren zu trainieren und die daraus resultierenden vorhergesagten mechanischen Eigenschaften des Trainingsdatensatzes mithilfe desselben Testdatensatzes zu verifizieren, wobei letztendlich jenes Lernverfahren ausgewählt wird, dessen Vorhersage die geringsten Abweichung zu den Beprobungsdaten $B_i$ der Testdatensätze aufweist.

Gemäß einer Ausgestaltung des erfindungsgemäßen Verfahrens zur Erstellung eines hybriden Modells umfassen die mechanischen Eigenschaften $J_f$ des ersten Walzguts sowie die Beprobungsdatensätze $B_i$, die ersten mechanischen Datensätze $C_i$ und die zweiten mechanischen Datensätze $S_i$ der weiteren Walzgüter jeweils eine Zugfestigkeit $Y_s$ und/oder eine Streckgrenze $T_s$ und/oder eine Bruchdehnung $A_l$.

Gemäß einer weiteren Ausgestaltung des erfindungsgemäßen Verfahrens zur Erstellung eines hybriden Modells wird vor der Durchführung des ersten Schrittes S1' der zu dem Produktionsdatensatz $P_i$ jeweils korrespondierende Beprobungsdatensatz $B_i$ von jedem der weiteren Walzgüter auf eine normierte Probengeometrie gemäß der Formel von Oliver transformiert.

Die mechanischen Eigenschaften eines Beprobungsdatensatzes $B_i$ einzelner Proben können nicht ohne Zusatzinformation in einem gemeinsamen Trainingsdatensatz zur Erzeugung eines statistischen Datenmodells zusammengefasst werden, da die Proben in der Regel eine jeweilige sorten- und dickenabhängige Geometrie aufweisen. Aufgrund dieser Geometrieabhängigkeit unterschiedlicher Proben lassen sich die in einem Beprobungsvorgang anhand einer Beprobungsvorrichtung ermittelten mechanischen Eigenschaften nicht für alle Sorten von Walzgütern (beispielsweise für Walzgüter aus unterschiedlichen Stahlsorten) übergreifend vergleichen. Um diese geometrie- und sortenbezogenen Unterschiede auszugleichen, ist es daher notwendig, die mechanischen Eigenschaften eines Beprobungsdatensatzes $B_i$ auf eine Normgeometrie zu beziehen.

Die Bruchdehnung $A_l$ in Prozent ist als der Quotient aus Längenänderung $\Delta L$ und einer Ausgangsmesslänge $L_0$ eines Probenstücks definiert. Dabei kann die Ausgangsmesslänge $L_0$ beispielsweise je nach Stahlsorte, Dicke, Norm oder Erzeugungsvorschrift unterschiedlich festgelegt sein. Die Ausgangsmesslänge kann ebenfalls variabel sein und von der Querschnittsfläche $S_0$ des Probenstücks abhängen. Sogenannte Proportionalproben weisen beispielsweise ein Verhältnis von $L_0 = 5,65 \cdot (S_0)^{0.5}$ auf. Bei rechteckigem Probenquerschnitt errechnet sich die Querschnittsfläche gemäß $S_0 = b_0 \cdot t_0$, wobei $b_0$ die Probenbreite und $t_0$ die Probendicke entsprechend ISO 6892-1:2019 Metallic materials - Tensile testing - Part 1: Method of test at room temperature, ASTM E8/E8M - 16ae1 Standard Test Methods for Tension Testing of Metallic Materials sind.

In einer gemäß der Norm ISO 2566-1 offenbarten Gleichung wird der Exponentialfaktor n mit dem Wert 0,4 für Stahl angegeben. Dies ist für moderne stahlbasierte Werkstoffe jedoch nicht vollumfänglich zutreffend bzw. zu ungenau, wie beispielsweise in Hanlon et al. (2015), Critical Assessment 10: Tensile elongation of strong automotive steels as function of testpiece geometry, Materials Science and Technology, 31:4, 385-388, DOI: 10.1179/1743284714Y.0000000707 dargelegt wird.

Weiterhin kann die Probenorientierung, d.h. die Ausrichtung zu einer Erzeugungsrichtung, unterschiedlich festgelegt sein. Die Orientierung kann in Längsrichtung (longitudinal, l) oder in Querrichtung (transversal, t) jeweils bezogen auf die Walzrichtung oder unter einem bestimmten Winkel erfolgen. Aufgrund der walzbedingten Anisotropie des metallurgischen Gefüges können die Ergebnisse voneinander abweichen.

Um daher alle gemessenen Werte der Bruchdehnung $A_l$ vergleichbar zu machen, werden sie beispielsweise mit Hilfe der Formel von Oliver

$$A_{y,o} = A_{x,o} \left[\frac{L_{0,x,o}}{\sqrt{S_{0,x,o}}}\right]^{n_{i,x\to y,o}} \left[\frac{\sqrt{S_{0,y,o}}}{L_{0,y,o}}\right]^{n_{i,x\to y,o}} \qquad (19)$$

von einer Probengeometrie x mit der Orientierung o, in der die Ermittlung der Bruchdehnung einer bestimmten Probe durchgeführt wurde, auf eine gemeinsame Probengeometrie y mit derselben Orientierung o umgerechnet. Die Orientierung der Probe bei der Beprobung kann beispielsweise horizontal oder longitudinal in Bezug auf die Walzrichtung ausgerichtet sein. Der Begriff 'Probengeometrie' bezeichnet dabei die ursprüngliche Länge der Probe (beispielsweise 50mm oder 80mm). In Formel (19) bezeichnen somit $A_{x,o}$ bzw. $A_{y,o}$ die Bruchdehnung in Bezug auf eine bestimmte Probengeometrie und Orientierung o, $L_{0,x/y,o}$ die ursprüngliche Probenlänge x oder y in der Orientierung o, $S_{0,x/y,o}$ den ursprünglichen Probenquerschnitt bei der Probenlänge x oder y in der Orientierung o und $n_{i,x\to y}$ einen individuellen, von der Art (z.B. der Stahlsorte), der Probengeometrie und der Orientierung o der jeweiligen Probe abhängigen Exponentialfaktor zur Umrechnung von der Probengeometrie x in der Orientierung o , in der die tatsächliche Beprobung durchgeführt wurde, eine gemeinsame Probengeometrie y mit derselben Orientierung o.

Da anhand der Formel (19) nur Werte von Proben mit derselben Orientierung o ineinander umgerechnet werden können, müssen die mechanischen Eigenschaften für die jeweilige Probenorientierung als separate mechanische Eigenschaften in die Beprobungsdaten $B_i$ aufgenommen werden (z.B. je ein Wert für die Bruchdehnung $A_l$ in longitudinaler und in transversaler Richtung). Alternativ können die jeweiligen mechanischen Eigenschaften jedoch auch von einer Probenorientierung mithilfe eines additiven Korrekturterms in eine andere Probenorientierung (z.B. gemäß $A_l = A_t + \Delta A$ mit $\Delta A$ als dem Korrekturterm) übergeführt werden, sofern ein Wert für die additive Korrekturterm bekannt, beispielsweise empirisch ermittelt worden ist.

Gemäß einer weiteren Ausgestaltung des erfindungsgemäßen Verfahrens zur Erstellung eines hybriden Modells werden die mechanischen Eigenschaften $J_f$ des ersten Walzguts und die ersten mechanischen Datensätze $C_i$ und die zweiten mechanischen Datensätze $S_i$ der weiteren Walzgüter jeweils für zumindest einen Walzgutabschnitt des ersten und der weiteren Walzgüter ermittelt. Die Vorteile und technischen Wirkungen korrespondieren dabei mit jenen des erfindungsgemäßen Verfahrens zur Bestimmung mechanischer Eigenschaften $J_f$ eines ersten Walzguts mit Hilfe eines hybriden Modells.

Analog zum erfindungsgemäßen Verfahren zur Bestimmung mechanischer Eigenschaften $J_f$ eines ersten Walzguts mit Hilfe eines hybriden Modells umfasst in einer weiteren Ausgestaltung des erfindungsgemäßen Verfahrens zur Erstellung eines hybriden Modells das Walzwerk N Walzgerüste $R_1,...,R_N$, L Kühlvorrichtungen $Q_1,...,Q_L$, Z Walzwerksabschnitte $A_1,...,A_Z$ und zumindest eine Haspelvorrichtung. Dabei umfassen die Produktionsdatensätze des ersten und der weiteren Walzgüter $P_f$, $P_i$ jeweils zumindest eine der folgenden Stell- oder Messgrößen für zumindest einen Walzgutabschnitt des ersten und der weiteren Walzgüter:

- einen oder mehrere Fremdelementanteile in dem Walzgutabschnitt,
- eine Dicke und/oder eine Breite des Walzgutabschnitts vor dem Eintritt in ein erstes Walzgerüst $R_1$ des Walzwerks,
- eine Dicke und/oder eine Breite des Walzgutabschnitts nach dem Austritt aus einem letzten Walzgerüst $R_N$ des Walzwerks,
- zumindest eine messtechnisch erfasste Temperatur des Walzgutabschnitts, bevorzugt eine unmittelbar vor dem Eintritt in das Walzwerk erfasste und/oder eine in einem der Walzwerksabschnitte $A_1,...,A_Z$ erfasste und/oder eine beim Aufhaspeln des ersten bzw. der weiteren Walzgüter erfasste Temperatur des Walzgutabschnitts,
- zumindest einen Walzspaltwert eines der Walzgerüste $R_1,...,R_N$ beim Durchtritt des Walzgutabschnitts durch das jeweilige Walzgerüst $R_1,...,R_N$ des Walzwerks,
- zumindest einen von einer der Kühlvorrichtungen $Q_1,...,Q_L$ des Walzwerks abgegebenen Kühlmittelstrom beim Durchtritt des Walzgutabschnitts durch den Wirkbereich der jeweiligen Kühlvorrichtung $Q_1,...,Q_L$, oder
- zumindest eine Geschwindigkeit des Walzgutabschnitts in einem der Walzwerksabschnitte $A_1,...,A_Z$ des Walzwerks.

Bevorzugt umfasst dabei der weitere Produktionsdatensatz $F_i$ für zumindest einen Walzgutabschnitt der weiteren Walzgüter jeweils zumindest eine modellierte Kühlrate $q_z$ und/oder zumindest einen modellierten Temperaturwert $T_z$ des jeweiligen Walzgutabschnitts beim Passieren von zumindest einem der Walzwerksabschnitte $A_1,...,A_Z$ des Walzwerks. Besonders bevorzugt umfassen dabei die Datensätze $K_i$ der weiteren Walzgüter Phasenanteile $p_h$ und/oder morphologische Kenngrößen $a_k$ verschiedener metallurgischer Phasen der weiteren Walzgüter bzw. zumindest eines Walzgutabschnitts der weiteren Walzgüter.

Wiederum korrespondieren Vorteile und technischen Wirkungen dabei mit jenen des erfindungsgemäßen Verfahrens zur Bestimmung mechanischer Eigenschaften $J_f$ eines ersten Walzguts mit Hilfe eines hybriden Modells.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung eines Ausführungsbeispiels, das in Zusammenhang mit den Figuren näher erläutert wird. Dabei zeigen:

Figur 1 (FIG 1) schematisch ein Warmwalzwerk umfassend eine zentralen Berechnungseinheit mit dem erfindungsgemäßen hybriden Modell,

Figur 2 (FIG 2) ein Ablaufdiagramm des erfindungsgemäßen Verfahrens zur Bestimmung mechanischer Eigenschaften eines ersten Walzguts anhand eines erfindungsgemäßen hybriden Modells,

Figur 3 (FIG 3) schematisch die Lage von Ein- und Ausgangsdaten des statistischen Datenmodells, und

Figur 4 (FIG 4) ein Ablaufdiagramm des erfindungsgemäßen Verfahrens zur Erstellung eines hybriden Modells.

Einander entsprechende Teile sind in den Figuren mit denselben Bezugszeichen versehen.

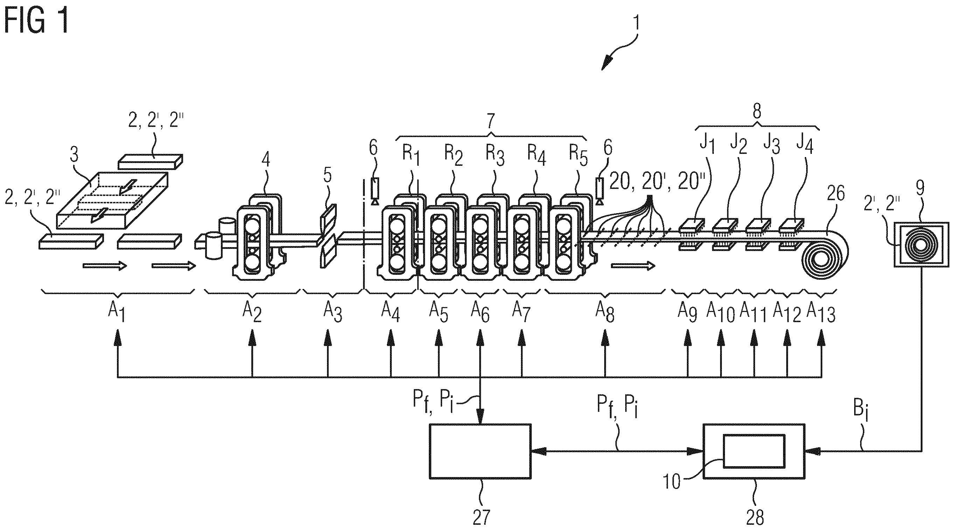

FIG 1 zeigt schematisch ein Warmwalzwerk 1. Das Warmwalzwerk 1 umfasst einen Erwärmungsofen 3, eine Vorwalzstraße 4, eine Schopfschere 5, eine Fertigwalzstraße 7, eine Kühlstrecke 8 und eine Haspelvorrichtung 26. Die Fertigwalzstraße 7 umfasst fünf Walzgerüste $R_1$, ... $R_5$, die Kühlstrecke 8 umfasst vier Kühlvorrichtungen $Q_1$ ... $Q_4$. In den Erwärmungsofen 3 wird ein Walzgut 2, 2', 2" in Form einer Bramme eingebracht und nach dem Erwärmen auf eine erhöhte Temperatur, beispielsweise auf 1100°C bis 1200°C, erwärmt und danach entlang der Produktionsrichtung des Warmwalzwerk 1 (in FIG 1 durch einen nach rechts verlaufenden horizontalen Pfeil dargestellt) in Richtung der Vorwalzstraße 4 transportiert.

In der Vorwalzstraße 4 erfolgt eine erste Dickenreduktion des Walzguts 2, 2', 2", das dabei von einem Brammenformat auf ein sogenanntes Vorband gewalzt wird. In Produktionsrichtung gesehen hinter der Vorwalzstraße 4 ist eine Schopfschere 5 angeordnet, mit deren Hilfe der Bandkopf bzw. das Bandende des Vorbands abgetrennt werden können, da diese Abschnitte des Vorbands in der Regel verschrottet werden müssen. Nach dem Passieren der Schopfschere 5 tritt das Vorband in die Fertigwalzstraße 7 ein, in der es auf eine vorgegebene Enddicke gewalzt wird. Weiterhin ist - bezogen auf die Produktionsrichtung des Warmwalzwerks 1 - unmittelbar vor dem ersten Walzgerüst $R_1$ und unmittelbar hinter dem letzten Walzgerüst $R_5$ jeweils eine Temperaturmessvorrichtung 6 in Form eines Pyrometers zur berührungslosen Erfassung einer Oberflächentemperatur des Walzguts 2, 2', 2" angeordnet.

Nach dem Austritt aus dem letzten Walzgerüst $R_5$ durchläuft das Walzgut 2, 2', 2" eine Kühlstrecke 8, in der es durch Aufbringen von Kühlmittel durch die Kühlvorrichtungen $Q_1$ ... $Q_4$ auf eine Haspeltemperatur gekühlt wird. Nach dem Verlassen des Wirkbereiches der letzten Kühlvorrichtung $Q_4$ wird das Walzgut 2, 2', 2" von einer Haspelvorrichtung 26 zu einem Bund aufgewickelt.

Entsprechend den genannten Gewerken wird das Warmwalzwerk 1 datentechnisch in einzelne Walzwerksabschnitte $A_1$ ... $A_Z$ mit Z=13 unterteilt. Symbolisch ist in FIG 1 der Walzwerksabschnitt $A_4$, der den Bereich des ersten Walzgerüsts $R_1$ und das einlaufseitige Pyrometer 6 umfasst, durch strichpunktierte Linien abgegrenzt. Der Produktionsprozess des Walzguts 2, 2', 2" in dem Warmwalzwerk 1 wird anhand der jeweiligen Produktionsparameter $P_f$, $P_i$ mit Hilfe einer Anlagensteuerung 27 gesteuert oder geregelt.

Die Anlagensteuerung 27 ist mit den Gewerken der einzelnen Walzwerksabschnitte $A_1$ ... $A_{13}$ datentechnisch verbunden, was in FIG 1 durch die bidirektionalen Pfeile symbolisiert ist. Aus Sicht der Anlagensteuerung 27 umfassen die Produktionsparameter $P_f$, $P_i$ sowohl Ausgangsdaten (z.B. Vorgabewerte für den Walzspalt der einzelnen Walzgerüste $R_1$ ... $R_5$), die an die jeweiligen Gewerke gesendet werden, als auch Eingangsdaten (wie z.B. eine Produktionsgeschwindigkeit im jeweiligen Walzwerksabschnitt oder Temperaturmesswerte), die die Anlagensteuerung 27 von den einzelnen Gewerken empfängt.

Datentechnisch wird das Walzgut 2, 2', 2" in einen, bevorzugt mehrere Walzgutabschnitte 20, 20', 20" von beispielsweise zwei bis 5 Metern Länge unterteilt. Die Walzgutabschnitte 20, 20', 20" werden während der Produktion in dem Walzwerk 1 wegverfolgt: dadurch ist es möglich, kurzzeitige Schwankungen der entsprechenden Prozessparameter $P_f$, $P_i$ einzelnen Walzgutabschnitten 20, 20', 20" zuzuordnen und über die Länge des Walzguts 2, 2', 2" zu erfassen.

Nach Abschluss des Produktionsvorganges werden von manchen der in dem Warmwalzwerk 1 produzierten Walzgütern 2', 2" mittels einer Beprobungsvorrichtung 9 Materialproben entnommen, deren mechanische Eigenschaften $B_i$ beispielsweise in einem Prüflabor gemessen und als Beprobungsdaten $B_i$ zusammen mit den korrespondierenden Produktionsdaten $P_i$ von der Anlagensteuerung 27 an eine zentrale Berechnungseinheit 28, die als Computer oder speicherprogrammierbare Steuerung (SPS) ausgestaltet ist und das erfindungsgemäße hybride Modell 10 umfasst, übermittelt werden. Auch Produktionsdaten $P_f$, von Walzgütern 2, die nicht beprobt werden, d.h. von denen keine Beprobungsdaten ermittelt werden, werden von der Anlagensteuerung 27 an die zentrale Berechnungseinheit 28 übermittelt, was durch einen bidirektionalen Pfeil zwischen der Anlagensteuerung 27 und der zentralen Berechnungseinheit 28 symbolisiert ist. In FIG 1 ist das erfindungsgemäße hybride Modell 10 mittels eines rechteckigen Symbols innerhalb der zentralen Berechnungseinheit 28 dargestellt. Die Beprobungsvorrichtung 9 und die zentrale Berechnungseinheit 28 sind dabei nicht Teil des Warmwalzwerks 1.

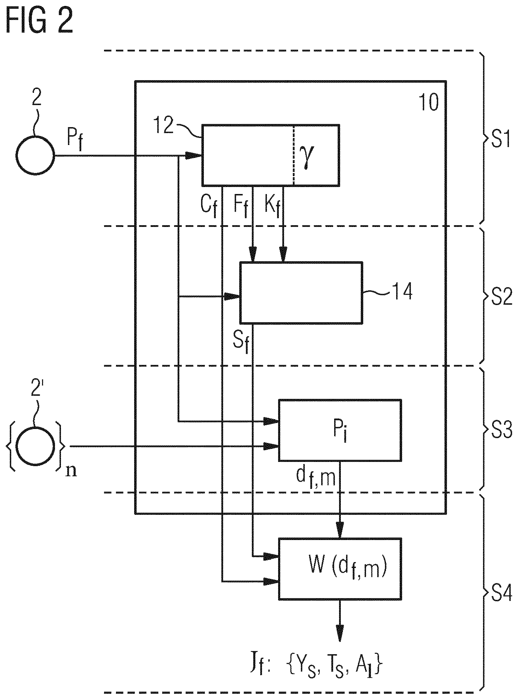

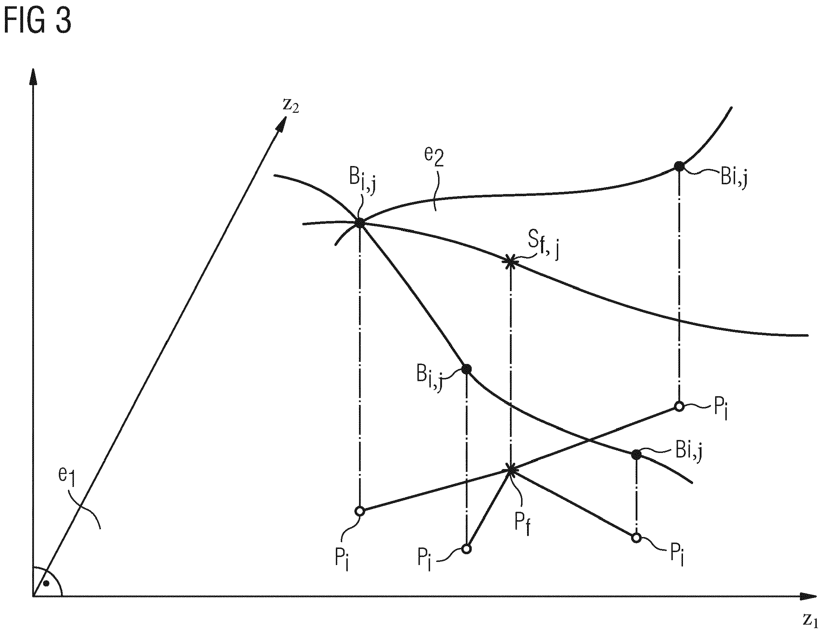

Figur 2 (FIG 2) zeigt ein Datenflussdiagramm des erfindungsgemäßen Verfahrens zur Bestimmung mechanischer Eigenschaften $J_f$ eines in einem Warmwalzwerk 1 produzierten ersten Walzguts 2 mit den Verfahrensschritten S1, S2, S3 und S4 anhand eines erfindungsgemäßen hybriden Modells 10. Das hybride Modell 10 umfasst Produktionsdatensätze $P_i$ von weiteren, in dem Walzwerk 1 produzierten Walzgütern 2', ein physikalisches Produktionsmodell 12 und ein trainiertes und validiertes statistisches Datenmodell 14 als strukturelle Bestandteile und ist in FIG 2 durch eine breite Linienstärke dargestellt. Diese strukturellen Bestandteile des hybriden Modells 10 sind in FIG 2 jeweils als rechteckige Symbole dargestellt. Daten des ersten bzw. der weiteren Walzgüter 2, 2' sind durch ein Kreissymbol bzw. ein Kreissymbol mit Mengenklammern gekennzeichnet. Die sequentiell ausgeführten Schritte S1 bis S4 sind in FIG 3 durch horizontale, gestrichelte Linien dargestellt. Pfeile symbolisieren einen Datenfluss, bei dem die zu einem Quellobjekt gehörenden bzw. von einem Quellobjekt stammenden Daten entlang der Pfeilrichtung an ein Zielobjekt übertragen werden.

Weiterhin können die Produktionsdatensätze $P_i$, das physikalische Produktionsmodell 12 und das Datenmodell 14 ihrerseits Datenstrukturen (beispielsweise eine zeitliche Abfolge einzelner Daten in den Produktionsdatensätzen $P_i$), interne Berechnungsvorschriften (beispielsweise eine Wärmeleitungsgleichung oder die empirischen metallurgischen Gleichungen des physikalischen Produktionsmodells 12), sowie Relationen zwischen verschiedenen Daten (beispielsweise Verknüpfungen zwischen Daten innerhalb des trainierten und validierten statistischen Datenmodells 14 bei der Ermittlung eines ersten mechanischen Datensatzes $C_f$ aus den Eingangsgrößen des Datenmodells 14) umfassen. Auch können die Produktionsdatensätze $P_i$, das physikalische Produktionsmodell 12 und das Datenmodell 14 jeweils weitere interne Parameter umfassen: dazu zählen beispielsweise die metallurgischen Modellparameter y oder interne Parameter des statistischen Datenmodells 14, mittels denen dessen Reaktion auf Eingangsgrößen festgelegt wird. Die genannten Datenstrukturen, interne Berechnungsvorschriften, Relationen zwischen Daten und internen Parameter sind im vorliegenden Ausführungsbeispiel jeweils als Daten bzw. programmtechnische Strukturen in der zentralen Berechnungseinheit 28 realisiert, sodass das erfindungsgemäße Verfahren zur Bestimmung mechanischer Eigenschaften $J_f$ eines ersten Walzguts 2 als Programm von der zentralen Berechnungseinheit 28 ausgeführt werden kann.

Im ersten Schritt S1 des erfindungsgemäßen Verfahrens wird ein Produktionsdatensatz $P_f$ eines ersten Walzguts 2 als Eingangsgröße an das physikalische Produktionsmodell 12 übermittelt. Daraus werden mit Hilfe des physikalischen Produktionsmodells 12 und mittels der metallurgischen Modellparameter y ein erster mechanischer Datensatz $C_f$, ein weiterer Produktionsdatensatz $F_f$ und ein metallurgischer Datensatz $K_f$ zu dem ersten Walzgut 2 ermittelt. Dafür werden von der zentralen Berechnungseinheit 28 physikalische Gleichungen (beispielsweise umfassend eine Wärmeleitungsgleichung und Gleichungen zur Bildung metallurgischer Phasen entsprechend den vorgenannten Gleichungen (1) bis (4)) mit entsprechenden Randbedingungen (beispielsweise entsprechend Gleichung (5)) gelöst. Gemäß dem vorliegenden Ausführungsbeispiel umfasst der weitere Produktionsdatensatz $F_f$ Temperaturen und Kühlraten des ersten Walzguts 2 während dessen Produktion in dem Warmwalzwerk 1, während der metallurgische Datensatz $K_f$ Phasenanteile $p_k$ und/oder morphologische Kenngrößen $a_k$ des ersten Walzguts 2 während dessen Produktion in dem Warmwalzwerk 1 umfasst.

Weiterhin werden von der zentralen Berechnungseinheit 28 aus dem Produktionsdatensatz $P_f$ des ersten Walzguts 2, aus den metallurgischen Modellparametern y und mittels empirischer metallurgischen Gleichungen (beispielsweise entsprechend den vorgenannten Gleichungen (6) bis (9)) die mechanischen Eigenschaften des ersten Walzguts 2 in Form des ersten mechanischen Datensatzes $C_f$ ermittelt. Der erste mechanische Datensatz $C_f$ umfasst gemäß Ausführungsbeispiel eine Zugfestigkeit $Y_s$, eine Streckgrenze $T_s$ und eine Bruchdehnung $A_l$ und wird für den vierten Schritt S4 des erfindungsgemäßen Verfahrens gespeichert, während der weitere Produktionsdatensatz $F_f$ und der metallurgische Datensatz $K_f$ dem statistischen Datenmodell 14 als zusätzliche Eingangsgrößen zugeführt werden.

Im zweiten Schritt S2 wird aus dem Produktionsdatensatz $P_f$, aus dem weiteren Produktionsdatensatz $F_f$ und dem metallurgischen Datensatz $K_f$ des ersten Walzguts 2 mit Hilfe des trainierten und validierten statistischen Datenmodells 14 der zweite mechanische Datensatz $S_f$ des ersten Walzguts 2 ermittelt und für den vierten Schritt S4 des erfindungsgemäßen Verfahrens gespeichert. Dabei umfasst der zweite mechanische Datensatz $S_f$ gemäß Ausführungsbeispiels wiederum eine Zugfestigkeit $Y_s$, eine Streckgrenze $T_s$ und eine Bruchdehnung $A_l$ des ersten Walzguts 2.

Im dritten Schritt S3 wird für den Produktionsdatensatz $P_f$ des ersten Walzguts 2 ein gemittelter normierter Abstandswert $d_{f,m}$ zu n Produktionsdatensätzen $P_i$ (in FIG 2 durch Mengenklammern mit Index n symbolisiert) der weiteren Walzgüter 2' bestimmt, was schematisch in FIG 3 dargestellt ist.

Im vierten Schritt S4 werden mit Hilfe des gemittelten normierten Abstandswertes $d_{f,m}$ die mechanischen Eigenschaften $J_f$ des ersten Walzguts 2 als gewichtetes Mittel aus dem ersten und zweiten mechanischen Datensatz $C_f$, $S_f$ des ersten Walzguts 2 ermittelt. Die Gewichtung erfolgt dabei komponentenweise, d.h. für jede mechanische Eigenschaft des ersten und zweiten mechanischen Datensatzes $C_f$ bzw. $S_f$ separat, wobei der Quotient $C_{f,j}/S_{f,j}$ aus der jeweiligen mechanischen Eigenschaft des zweiten und ersten mechanischen Datensatzes $S_f$, $C_f$ proportional zum gemittelten normierten Abstandswert $d_{f,m}$ gewichtet wird. Dies bedeutet, dass die betreffende mechanische Eigenschaft aus dem zweiten mechanischen Datensatz $S_f$ im Verhältnis zu jener aus dem ersten mechanischen Datensatz $C_f$ umso stärker gewichtet wird, je kleiner der Wert von $d_{f,m}$ ist. Die Gewichtung erfolgt anhand der vorgenannten Formel (10), wobei eine geeignete Gewichtungsfunktion $w(d_{f,m})$ wie beispielsweise jene von Formel (11) verwendet wird.

FIG 3 zeigt schematisch anhand eines vereinfachten statistischen Datenmodells 14, dessen Eingangsgrößen (entsprechend den Produktionsdatensätzen $P_f$ bzw. $P_i$, den weiteren Produktionsdatensätzen $F_f$ bzw. $F_i$ und den metallurgischen Datensätzen $K_f$ bzw. $K_i$ des ersten und der weiteren Walzgüter 2 bzw. 2') insgesamt nur zwei standardisierte Produktionsparameter $z_1$ und $z_2$ umfasst, die Lage eines standardisierten Produktionsdatensatzes $P_f$ eines ersten Walzguts 2 in Relation vier standardisierten Produktionsdatensätzen $P_i$ weiterer Walzgüter 2'. In einem realistischen Anwendungsfall ist die Anzahl M von Eingangsgrößen (entsprechend den Produktionsdatensätzen $P_f$ bzw. $P_i$, den weiteren Produktionsdatensätzen $F_f$ bzw. $F_i$ und den metallurgischen Datensätzen $K_f$ bzw. $K_i$ des ersten und der weiteren Walzgüter 2 bzw. 2') des statistischen Datenmodells 14 größer als 2, beispielsweise 20 oder 50. Eine zu FIG 3 korrespondierende Darstellung eines realistischen statistischen Datenmodells 14 würde dementsprechend eine mathematische Abbildung von einer M-dimensionalen Mannigfaltigkeit auf eine gekrümmte M-dimensionale Mannigfaltigkeit umfassen.

Bei der in FIG 3 dargestellten mechanischen Eigenschaft $S_{f,j}$ handelt es sich um einen Wert (Index j) aus dem zweiten mechanischen Datensatz $S_f$. Die Produktionsparameter des ersten Walzguts 2 und der weiteren Walzgüter 2' werden dabei gemäß der vorgenannten Formeln (13a) bzw. (13b) auf dimensionslose und standardisierte Werte $z_1$ bzw. $z_2$ umgerechnet, wofür zunächst die jeweils betragsmäßig kleinsten und größten Werte $x_{1(min)}$, $x_{2,min}$, $x_{1,max}$, $x_{2,max}$ der Produktionsparameter aller weiteren Walzgüter 2' ermittelt werden.

Die beiden standardisierten Produktionsparameter $z_1$ und $z_2$ spannen in FIG 3 eine horizontal verlaufende mathematische Ebene $e_2$ auf, in der die Lage der standardisierten Produktionsparameter $P_f$ des ersten Walzguts (Stern) und von Produktionsparametern $P_i$ vier weiterer Walzgüter 2' (offene Kreise) gekennzeichnet ist. Aus der relativen Lage der standardisierten Produktionsparameter in der Ebene $e_1$ zueinander lässt sich beispielsweise anhand von Formel (12) ein normierter Abstandswert $d_{f,i}$ des Produktionsdatensatzes $P_f$ des ersten Walzguts 2 zum jeweiligen Produktionsdatensatz $P_i$ eines der weiteren Walzgüter 2' ermitteln, wobei für das vereinfachte Beispiel von FIG 3 aufgrund der lediglich zwei Eingangsparameter z1 und z2 in Formel (12) der Wert M = 2 gewählt werden muss. Die in FIG 3 eingezeichneten Verbindungslinien zwischen den standardisierten Produktionsparametern $P_f$ des ersten Walzguts 2 und der standardisierten Produktionsparameter $P_i$ der weiteren Walzgüter 2' stellen eine Alternative zu Formel (12) in Form der euklidischen Norm innerhalb der Ebene $e_1$ zur Berechnung eines normierten Abstandswert $d_{f,i}$ des Produktionsdatensatzes $P_f$ des ersten Walzguts 2 zum jeweiligen Produktionsdatensatz $P_i$ eines weiteren Walzguts 2' dar.

Nach der Berechnung der normierten Abstandswerte $d_{f,i}$ wird durch arithmetische Mittelwertbildung aus den n kleinsten normierten Abstandswerten $d_{f,i}$ der gemittelte normierte Abstandswert $d_{f,m}$ des Produktionsdatensatzes $P_f$ ersten Walzguts 2 zu den Produktionsdatensätzen $P_i$ der weiteren Walzgüter 2' ermittelt, wobei die Anzahl n 0,1 bis 0,5 Prozent der Gesamtzahl der weiteren Walzgüter 2' umfasst.

In FIG 3 ist weiterhin entlang der vertikal verlaufenden Achse, die normal zur Ebene $e_1$ verläuft, zu jedem der vier dargestellten Beprobungsdatensätze $P_i$ der weiteren Walzgüter 2' jeweils der Wert einer mechanischen Eigenschaft mit Index j aus dem zugehörigen Beprobungsdatensatz $B_i$ als Vollkreis eingezeichnet. Diese Datenpunkte aus den Beprobungsdatensätzen $B_i$ approximieren eine weitere, im Allgemeinen gekrümmte Ebene $e_2$, die in FIG 3 durch gekrümmt verlaufende Linien angedeutet ist. "Approximieren" bedeutet in diesem Zusammenhang, dass die Datenpunkte aus den Beprobungsdatensätzen $B_i$ nicht notwendigerweise exakt auf der Ebene $e_2$ liegen, sondern gegebenenfalls auch in einem geringen lokalen Normalabstand dazu positioniert sein können, weil die die mathematische Ebene $e_2$ im konkreten Beispiel von FIG 3 eine Regressionsebene darstellt. Die Regressionsebene $e_2$ stellt im Rahmen der Vorhersagegenauigkeit des statistischen Datenmodells 14 eine Annäherung an die Datenpunkte der Beprobungsdatensätze $B_i$ der weiteren Walzgüter 2' dar, mit denen das statistischen Datenmodell 14 trainiert worden ist.

Entlang der vertikalen Achse ist in FIG 3 oberhalb des standardisierten Produktionsdatensatzes $P_f$ des ersten Walzguts 2 der von dem statistischen Datenmodell 14 vorhergesagte Wert $S_{f,j}$ der mechanischen Eigenschaft aus dem ersten mechanischen Datensatz $S_f$ des ersten Walzguts 2 dargestellt: dieser berechnete Wert ersetzt einen physischen Beprobungsvorgang des ersten Walzguts 2 und liegt in der Regel 'in der Nähe' der Ebene $e_2$ - dies bedeutet: wenn das statistische Datenmodell 14 eine hundertprozentige Vorhersagegenauigkeit aufweist, so ist der Datenpunkt $S_{f,j}$ auch Element der Ebene $e_2$. Da jedoch in dem betrachteten Bereich der Produktionsparameter $z_1$ und $z_2$ nur eine endliche Anzahl von Daten weiterer Walzgütern 2' zum Trainieren des statistische Datenmodell 14 zur Verfügung stehen, liegt der von dem statistischen Datenmodell vorhergesagte Wert $S_{f,j}$ - abhängig vom gemittelten normierten Abstandswert $d_{f,m}$ - in einem bestimmten lokalen Normalabstand zur Ebene $e_2$ entfernt. In anderen Worten ausgedrückt: in stark besetzten Bereichen der Trainingsdaten des statistischen Datenmodells 14 (resultierend in einem kleinen Wert von $d_{f,m}$ für ein erstes Walzgut 2), in denen eine hohe Zahl von Trainingsdatensätzen vorhanden ist, liegt der von dem statistischen Datenmodell 14 vorhergesagte Wert $S_{f,j}$ in der Regel näher an der durch das Ensemble der Beprobungsdaten $B_i$ approximierten Ebene $e_2$ als in dünn besetzten Bereichen von Trainingsdaten (großer Wert von $d_{f,m}$).

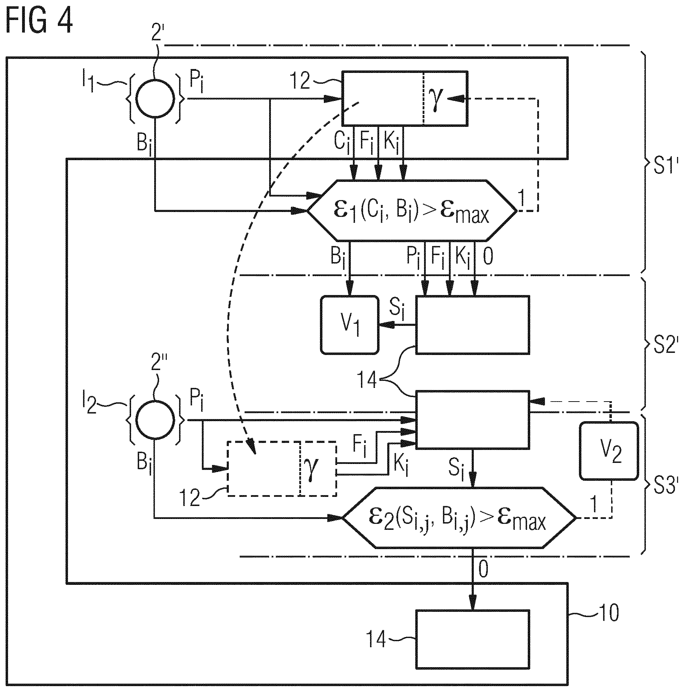

FIG 4 zeigt ein Datenflussdiagramm des erfindungsgemäßen Verfahrens zur Erstellung eines hybriden Modells 10. Das erfindungsgemäße Verfahren umfasst drei sequentielle - d.h. hintereinander auszuführende - Schritte S1' bis S3'. Analog zu FIG 2 ist das hybride Modell 10 mit breiter Linienstärke dargestellt und umfasst Produktionsdatensätze $P_i$ von weiteren Walzgütern 2', ein physikalisches Produktionsmodell 12 mit einem Satz von metallurgischen Modellparametern y und ein statistisches Datenmodell 14. Die weiteren Walzgüter 2' sowie Walzgüter 2" bilden eine erste Menge $I_1$ bzw. eine zweite enge $I_2$ und sind in FIG 4 jeweils durch ein Kreissymbol mit Mengenklammern dargestellt. Die sequentiellen Schritte des Verfahrens werden wie in FIG 2 durch horizontale, gestrichelte Linien repräsentiert; durchgezogene Pfeile symbolisieren wiederum entsprechende Datenflüsse.

Die Walzgüter der ersten und zweiten Menge $I_1$, $I_2$ sind jeweils in dem Warmwalzwerk 1, zu dem das hybride Modell 10 erstellt wird, produziert und anschließend physisch beprobt worden, sodass entsprechende Produktionsdaten $P_i$ und Beprobungsdaten $B_i$ verfügbar sind. Diese Daten sind beispielsweise gemäß FIG 1 an die zentrale Auswerteeinheit 28 übertragen worden, in der auch das hybride Modell 10 erstellt wird.

Ebenfalls in Analogie zu FIG 2 sind im vorliegenden Ausführungsbeispiel zur Erstellung eines hybriden Modells 10 interne Datenstrukturen, interne Berechnungsvorschriften, Relationen zwischen Daten und interne Parameter jeweils als Daten bzw. programmtechnische Strukturen in der zentralen Berechnungseinheit 28 realisiert, sodass das erfindungsgemäße Verfahren zur Erstellung eines hybriden Modells 10 wiederum als Programm von der zentralen Berechnungseinheit 28 ausgeführt werden kann.

Im ersten Schritt S1' wird das physikalische Produktionsmodell 12 zumindest einmal durchlaufen: dabei wird zu jedem der Produktionsdatensätze $P_i$ der weiteren Walzgüter 2' der erste mechanische, der weitere Produktions- und der metallurgische Datensatz $C_i$, $F_i$, bzw. $K_i$ ermittelt.

Nach jeder Ermittlung dieser Datensätze wird mit Hilfe einer ersten Maßfunktion $\varepsilon_1$ komponentenweise, d.h. für alle einzelnen mechanischen Eigenschaften in den weiteren mechanischen Datensätzen $C_i$, ein Maß für die Abweichung der ermittelten ersten mechanischen Datensätze $C_i$ von den Beprobungsdatensätzen $B_i$ der weiteren Walzgüter 2' ermittelt, wobei als erste Maßfunktion $\varepsilon_1$ gemäß vorliegendem Ausführungsbeispiel der Ausdruck der vorgenannten Formel (16) oder (17) verwendet wird. Wenn der dabei ermittelte Wert der ersten Maßfunktion ($\varepsilon_1$) für mindestens eine mechanische Eigenschaft in den ersten mechanischen Datensätzen $C_i$ der weiteren Walzgüter 2' größer als eine vorgegebene Schranke $\varepsilon_{max}$ ist, wird das physikalischen Produktionsmodell 12 adaptiert, indem dessen metallurgischen Modellparameter y variiert werden: dies ist in FIG 4 durch den mit '1' gekennzeichneten strichlierten Pfeil der ersten Programmverzweigung dargestellt, dessen Pfeilspitze in das Rechtecksymbol des physikalischen Produktionsmodells 12 hineinragt.

Daraufhin wird das physikalischen Produktionsmodell 12 erneut durchlaufen, was auch als eine 'weitere Iteration' bezeichnet wird. Dabei werden die ersten mechanischen, weiteren Produktions- und metallurgischen Datensätze $C_i$, $F_i$ und $K_i$ durch Lösen der entsprechenden Gleichungen des physikalischen Produktionsmodells 12 - in diesem Fall jedoch mit den geänderten metallurgischen Modellparametern y - erneut ermittelt, woraufhin wiederum anhand der ersten Maßfunktion $\varepsilon_1$ eine Überprüfung der Abweichung der Werte der neu ermittelten ersten mechanischen Datensätzen $C_i$ von den entsprechenden Werten der Beprobungsdatensätze $B_i$ erfolgt.

In dem Fall, dass nach einem Durchlauf des physikalischen Produktionsmodells 12 Wert der ersten Maßfunktion $\varepsilon_1$ für alle mechanischen Eigenschaften $C_{i,j}$ (j bezeichnet einen einzelnen Wert innerhalb eines mechanischen Datensatzes $C_i$) in den ersten mechanischen Datensätzen $C_i$ der weiteren Walzgüter 2' kleiner oder gleich der Schranke $\varepsilon_{max}$ ist, werden die Produktionsdatensätze $P_i$ der weiteren Walzgüter 2' zusammen mit den dazu korrespondierenden weiteren Produktions- und metallurgischen Datensätzen $F_i$, $K_i$, die in der zuletzt durchgeführten Iteration ermittelten wurden, dem statistischen Datenmodell 14 als Eingangsgrößen und die dazu korrespondierenden Beprobungsdatensätze $B_i$ als Zielgrößen übermittelt. Dieser Fall wird in FIG 4 durch die mit '0' gekennzeichneten Pfeile der ersten Programmverzweigung repräsentiert.

Im zweiten Schritt S2' wird das statistische Datenmodell (14) anhand der übermittelten Eingangs- und Zielgrößen mit Hilfe eines ersten maschinellen Lernverfahrens $V_1$ (in FIG 4 durch ein Quadrat mit gerundeten Ecken dargestellt) trainiert. Maschinelle Lernverfahren sind dem Fachmann bekannt. Dabei werden interne Parameter des statistischen Datenmodells 14 entsprechend festgelegt: das derart trainierte statistische Datenmodell ist in der Lage, aus einem Satz von Eingangsparametern (beispielsweise aus den Produktionsparametern und weiteren Produktionsparametern eines Walzguts) die dazu korrespondierenden mechanischen Eigenschaften ohne Beprobungsvorgang vorherzusagen. In dem Analogiebeispiel von FIG 3 bedeutet dies, dass das trainierte statistische Datenmodell 14 in der Lage ist, zu jeder mechanischen Eigenschaft die Ebene $e_1$ (entsprechend den standardisierten Produktionsparametern $z_1$, $z_2$) auf die - im allgemeinen gekrümmte - Ebene $e_2$ abzubilden.

Im anschließenden dritten Schritt S3' wird das trainierte statistische Datenmodell 14 anhand von Produktionsdatensätzen $P_i$ und Beprobungsdatensätzen $B_i$ von Walzgütern 2" aus einer zweiten Menge $I_2$ validiert. Die zweiten Menge $I_2$ ist zur ersten Menge $I_1$ disjunkt, was bedeutet, dass keines der Walzgüter, dessen Produktions- und Beprobungsdaten Pi, Bi zum Trainieren des statistischen Datenmodells 14 verwendet worden sind, für die Validierung des trainierten statistischen Datenmodells 14 herangezogen werden.

In dem vorliegenden Ausführungsbeispiel des erfindungsgemäßen Verfahrens werden für die Validierung die

Produktionsdatensätze $P_i$ der Walzgüter der zweiten Menge $I_2$ dem physikalischen Produktionsmodell 12 mit den metallurgischen Modellparametern y, die in der zuletzt durchgeführten Iteration verwendet worden sind, zugeführt: dies ist in FIG 4 durch den strichlierten Pfeil auf das ebenfalls strichliert dargestellte physikalische Produktionsmodell 12 im dritten Verfahrensschritt S3' dargestellt. Mittels eines einzigen Durchlaufs des physikalischen Produktionsmodells 12 werden die weiteren Produktionsdatensätze $F_i$ und die metallurgischen Datensätze $K_i$ zu den Walzgütern der zweiten Menge $I_2$ ermittelt und diese anschließend zusammen mit den dazu korrespondierenden Produktionsdatensätzen $P_i$ dem trainierten statistischen Datenmodell 14 als Eingangsgrößen zugeführt. Daraufhin werden daraus von dem statistischen Datenmodell 14 dazu korrespondierende zweite mechanische Datensätze $S_i$ für die Walzgüter der zweiten Menge $I_2$ ermittelt.

Anhand einer zweiten Maßfunktion $\varepsilon_2$, die im vorliegenden Ausführungsbeispiel durch die vorgenannte Formel (18) gegeben ist, wird ein Maß für eine Abweichung der einzelnen Größen in den mechanischen Datensätze $S_i$ zu den jeweils dazu korrespondierenden Größen in den Beprobungsdatensätzen $B_i$ der Walzgüter der zweiten Menge $I_2$ ermittelt. Falls jeder anhand der zweiten Maßfunktion $\varepsilon_2$ ermittelte Wert kleiner oder gleich einer vorgegebene Schranke $\varepsilon_{max}$ ist, wird das statistische Datenmodell 14 mit den im Rahmen des Trainierens bestimmten internen Parametern in das hybride Modell 10 übernommen (in FIG 4 durch den mit '0' gekennzeichneten Pfeil der zweiten Programmverzweigung dargestellt). Bei der Schranke $\varepsilon_{max}$ kann es sich um denselben Wert handeln, der auch bei der Adaption des physikalischen Produktionsmodells im ersten Verfahrensschritt S1' verwendet worden ist.

In dem Fall, dass einer der anhand der zweiten Maßfunktion $\varepsilon_2$ ermittelten Werte größer als die vorgegebene Schranke $\varepsilon_{max}$ ist, wird das statistische Datenmodell weiter adaptiert, indem im vorliegenden Ausführungsbeispiel des erfindungsgemäßen Verfahrens das statistische Datenmodell 14 (bzw. jedes Teildatenmodell) anhand eines zweiten maschinellen Lernverfahren $V_2$, das unterschiedlich zu dem ersten zu dem ersten maschinellen Lernverfahren $V_1$ ist, trainiert wird und der zweite Verfahrensschritt S2' wird wiederholt: dieser Fall ist in FIG 4 durch den mit '1' gekennzeichneten strichlierten Pfeil der zweiten Programmverzweigung angedeutet. Im Gegensatz zum vorgenannten 'ersten' maschinellen Lernverfahren kommt im Rahmen der Erfindung das 'zweite' maschinelle Lernverfahren nur im Falle einer nicht erfolgreichen Validierung des statistischen Datenmodells 14 (bzw. eines der Teildatenmodelle) zum Einsatz.

Alternativ oder zusätzlich können im Falle einer nicht erfolgreichen Validierung (d.h. $\varepsilon_2 \geq \varepsilon_{max}$) auch die erste und die zweite Menge $I_1$, $I_2$ an Walzgütern, deren Produktions- und Beprobungsdaten $P_i$, $B_i$ zum Trainieren und Validieren des statistischen Datenmodells 14 verwendet werden, geändert werden (in FIG 4 nicht dargestellt). Wiederum kann zur Validierung der einzelnen Teildatenmodelle jeweils ein- und dasselbe zweite maschinelle Lernverfahren $V_2$ verwendet werden, das lediglich zum ersten maschinellen Lernverfahren $V_1$ des jeweiligen Teildatenmodells unterschiedlich ist. Alternativ können sich die zweiten maschinellen Lernverfahren $V_2$ zur Validierung der einzelnen Teildatenmodelle auch untereinander unterscheiden.

Bezugszeichenliste

| | |
|---|---|
| 1 | Walzwerk, Warmwalzwerk |
| 2, 2', 2" | Walzgut |
| 3 | Erwärmungsofen |
| 4 | Vorwalzstraße |
| 5 | Schopfschere |
| 6 | Temperaturmessvorrichtung, Pyrometer |
| 7 | Fertigwalzstraße |
| 8 | Kühlstrecke |
| 9 | Beprobungsvorrichtung |
| 10 | hybrides Modell |
| 12 | physikalisches Produktionsmodell |
| 14 | statistisches Datenmodell |
| 20, 20', 20" | Walzgutabschnitt |
| 26 | Haspelvorrichtung |
| 27 | Anlagensteuerung |
| 28 | zentrale Auswerteeinheit |

| | |
|---|---|
| $a_k$ | morphologische Kenngröße |
| $A_1 \ldots A_{13}$ | Walzwerksabschnitt |
| $B_i$ | Beprobungsdatensatz |
| $B_{i,j}$ | Wert aus dem Beprobungsdatensatz |

| $C_f$, $C_i$ | erster mechanischer Datensatz |
|---|---|
| $C_{f,j}$, $C_{i,j}$ | Einzelwert in erstem mechanischem Datensatz |
| $d_{f,i}$ | normierter Abstandswert |
| $d_{f,m}$ | gemittelter normierter Abstandswert |
| $\delta_j$ | Funktionsparameter |
| $e_1$, $e_2$ | Ebene |
| $\varepsilon_1$, $\varepsilon_2$ | Maßfunktion |
| $\varepsilon_{max}$ | Schranke |
| $F_f$, $F_i$ | weiterer Produktionsdatensatz |
| $\gamma$ | metallurgische Modellparameter |
| $I_1$, $I_2$ | Menge |
| $J_f$ | mechanische Eigenschaften |
| $J_{f,j}$ | einzelne mechanische Eigenschaft |
| $K_f$, $K_i$ | metallurgischer Datensatz |
| $\lambda_j$ | Funktionsparameter |
| $p_k$ | Phasenanteil |
| $P_f$, $P_i$ | Produktionsdatensatz |
| $q_z$ | Kühlrate |
| $Q_1 \ldots Q_4$ | Kühlvorrichtung |
| $R_1 \ldots R_5$ | Walzgerüst |
| S1 ... S5 | Verfahrensschritt |
| S1' ... S3' | Verfahrensschritt |
| $S_f$, $S_i$ | zweiter mechanischer Datensatz |
| $S_{f,j}$ | Wert aus dem zweiten mechanischen Datensatz |
| $T_z$ | Temperaturwert |
| $V_1$, $V_2$ | maschinelles Lernverfahren |
| $w_i$ | Gewichtungsfaktor |
| $w_j (d_{f,m})$ | Gewichtungsfunktion |
| $x_{k,f}$, $x_{k,i}$ | Komponente in Produktionsdatensatz |
| $x_{k,min}$ | minimaler Wert Komponente k |
| $x_{k,max}$ | maximaler Wert Komponente |
| $z_{k,f}$, $z_{k,i}$ | standardisierter Wert in Produktionsdatensatz |

## Patentansprüche

1. Verfahren zur Bestimmung mechanischer Eigenschaften ($J_f$) eines in einem Walzwerk (1) produzierten ersten Walzguts (2) mit Hilfe eines hybriden Modells (10), ausgehend von einem Produktionsdatensatz ($P_f$) des ersten Walzguts (2), das hybride Modell (10) umfassend

- Produktionsdatensätze ($P_i$) von weiteren in dem Walzwerk (1) produzierten Walzgütern (2') aus einer erste Menge ($I_1$), zu denen jeweils korrespondierende Beprobungsdatensätze ($B_i$), umfassend die nach der Produktion in dem Walzwerk (1) mittels einer Beprobungsvorrichtung (9) ermittelten mechanischen Eigenschaften der weiteren Walzgüter (2'), vorliegen,
- ein physikalisches Produktionsmodell (12) umfassend einen Satz von metallurgischen Modellparametern (y), zur Ermittlung

-- eines ersten mechanischen Datensatzes ($C_f$), umfassend die mechanischen Eigenschaften ($J_f$) des ersten Walzguts (2) nach der Produktion in dem Walzwerk (1),
-- eines weiteren Produktionsdatensatzes ($F_f$), umfassend weitere Produktionsdaten des ersten Walzguts (2) während der Produktion in dem Walzwerk (1),
-- und eines metallurgischen Datensatzes ($K_f$), umfassend metallurgische Eigenschaften des ersten Walzguts (2) während der Produktion in dem Walzwerk (1), und

- ein trainiertes und validiertes statistisches Datenmodell (14) zur Ermittlung eines zweiten mechanischen Datensatzes ($S_f$), umfassend die mechanischen Eigenschaften des ersten Walzguts (2) nach der Produktion in dem Walzwerk (1),

wobei das Verfahren einen ersten, zweiten, dritten und vierten Schritt (S1, S2, S3, S4) aufweist, wobei

- im ersten Schritt (S1) aus dem Produktionsdatensatz ($P_f$) des ersten Walzguts (2) mittels des physikalischen Produktionsmodells (12) der erste mechanische Datensatz ($C_f$), der weitere Produktionsdatensatz ($F_f$) und der metallurgische Datensatz ($K_f$) des ersten Walzguts (2) ermittelt werden,
- im zweiten Schritt (S2) aus dem Produktionsdatensatz ($P_f$), dem weiteren Produktionsdatensatz ($F_f$) und dem metallurgischen Datensatz ($K_f$) des ersten Walzguts (2) mittels des statistischen Datenmodells (14) der zweite mechanische Datensatz ($S_f$) des ersten Walzguts (2) ermittelt wird,
- im dritten Schritt (S3) für den Produktionsdatensatz ($P_f$) des ersten Walzguts (2) ein gemittelter normierter Abstandswert ($d_{f,m}$) zu einer Anzahl (n) an Produktionsdatensätzen ($P_i$) der weiteren Walzgüter (2') bestimmt wird, und
- im vierten Schritt (S4) mit Hilfe des gemittelten normierten Abstandswertes ($d_{f,m}$) die mechanischen Eigenschaften ($J_f$) des ersten Walzguts (2) als gewichtetes Mittel aus dem ersten und zweiten mechanischen Datensatz ($C_f$, $S_f$) ermittelt werden, wobei die Quotienten aus den einander entsprechenden Werten des zweiten und ersten mechanischen Datensatzes ($S_f$, $C_f$) proportional zum gemittelten normierten Abstandswert ($d_{f,m}$) gewichtet werden.

2. Verfahren nach Anspruch 1, wobei die mechanischen Eigenschaften ($J_f$) des ersten Walzguts (2) eine Zugfestigkeit ($Y_s$) und/oder eine Streckgrenze ($T_s$) und/oder eine Bruchdehnung ($A_l$) umfassen.

3. Verfahren nach Anspruch 1 oder 2, wobei der gemittelte normierte Abstandswert ($d_{f,m}$) aus (n) normierten Abstandswerten ($d_{f,i}$) des Produktionsdatensatzes ($P_f$) des ersten Walzguts (2) zu jeweils einem Produktionsdatensatz ($P_i$) eines der weiteren Walzgüter (2') gebildet wird, und wobei die Anzahl (n) 0,1 bis 0,5 Prozent der Trainingsdatensätze statistischen Datenmodells (14) umfasst.

4. Verfahren nach einem der vorangegangenen Ansprüche, wobei die mechanischen Eigenschaften ($J_f$) des ersten Walzguts (2) für zumindest einen Walzgutabschnitt (20) des ersten Walzguts (2) ermittelt werden.

5. Verfahren nach Anspruch 4, das Walzwerk (1) aufweisend (N) Walzgerüste ($R_1$,...,$R_N$), (L) Kühlvorrichtungen ($Q_1$,...,$Q_L$), (Z) Walzwerksabschnitte ($A_1$,...,$A_Z$) und zumindest eine Haspelvorrichtung (26), wobei die Produktionsdatensätze ($P_f$, $P_i$) des ersten und der weiteren Walzgüter (2, 2') jeweils zumindest eine der folgenden Stell- oder Messgrößen für zumindest einen Walzgutabschnitt (20, 20') des ersten und der weiteren Walzgüter (2, 2') umfassen:

- einen oder mehrere Fremdelementanteile in dem Walzgutabschnitt (20, 20'),
- eine Dicke und/oder eine Breite des Walzgutabschnitts (20, 20') vor dem Eintritt in ein erstes Walzgerüst ($R_1$),
- eine Dicke und/oder eine Breite des Walzgutabschnitts (20, 20') nach dem Austritt aus einem letzten Walzgerüst ($R_N$),
- zumindest eine messtechnisch erfasste Temperatur des Walzgutabschnitts (20, 20'),
- zumindest einen Walzspaltwert eines der Walzgerüste ($R_1$,...,$R_N$) beim Durchtritt des Walzgutabschnitts (20, 20') durch das entsprechende Walzgerüst ($R_1$,...,$R_N$),
- zumindest einen von einer der Kühlvorrichtungen ($Q_1$,...,$Q_L$) abgegebenen Kühlmittelstrom beim Durchtritt des Walzgutabschnitts (20, 20') durch den Wirkbereich der entsprechenden Kühlvorrichtung ($Q_1$,...,$Q_L$), oder
- zumindest eine Geschwindigkeit des Walzgutabschnitts (20, 20') in einem der Walzwerksabschnitte ($A_1$,...,$A_Z$) .

6. Verfahren nach Anspruch 4 oder 5, wobei der weitere Produktionsdatensatz ($F_f$) für zumindest einen Walzgutabschnitt (20) des ersten Walzguts (2) zumindest eine modellierte Kühlrate ($q_z$) und/oder zumindest einen modellierten Temperaturwert ($T_z$) des Walzgutabschnitts beim Passieren von zumindest einem der Walzwerksabschnitte ($A_1$,...,$A_Z$) des Walzwerks (1) umfasst.

7. Verfahren nach einem der Ansprüche 4 bis 6, wobei die metallurgischen Eigenschaften des metallurgischen Datensatzes ($K_f$) Phasenanteile ($p_k$) und/oder morphologische Kenngrößen ($a_k$) von verschiedenen metallurgischen Phasen zumindest eines Walzgutabschnitts (20) des ersten Walzguts (2) umfassen.

8. Verfahren nach einem der Ansprüche 4 bis 7, wobei in einem fünften Schritt (S5) die ermittelten mechanischen Eigenschaften ($J_f$) des ersten Walzguts (2) einer Abkühlungskorrektur unterzogen werden, bei der Karbid- und Nitridausscheidungen in dem ersten Walzgut (2) während des Abkühlens nach der Produktion in dem Walzwerk (1) berücksichtigt werden.

9. Verfahren zur Erstellung eines hybriden Modells (10) für die Bestimmung mechanischer Eigenschaften ($J_f$) eines in einem Walzwerk (1) produzierten ersten Walzguts (2), das hybride Modell (10) umfassend

- Produktionsdatensätze ($P_i$) von weiteren in dem Walzwerk (1) produzierten Walzgütern (2') aus einer erste Menge ($I_1$), zu denen jeweils korrespondierende Beprobungsdatensätze ($B_i$), umfassend die nach der Produktion in dem Walzwerk (1) mittels einer Beprobungsvorrichtung (9) ermittelten mechanischen Eigenschaften der weiteren Walzgüter (2'), vorliegen,
- ein physikalisches Produktionsmodell (12) mit einem Satz von metallurgischen Modellparametern (y) zur Ermittlung

-- eines ersten mechanischen Datensatzes ($C_i$), umfassend die mechanischen Eigenschaften nach der Produktion in dem Walzwerk (1),
-- eines weiteren Produktionsdatensatzes ($F_i$), umfassend weitere Produktionsdaten während der Produktion in dem Walzwerk (1),
-- und eines metallurgischen Datensatzes ($K_i$), umfassend metallurgische Eigenschaften während der Produktion in dem Walzwerk (1),
für jedes der weiteren Walzgüter (2'), und

- ein statistisches Datenmodell (14) zur Ermittlung eines zweiten mechanischen Datensatzes ($S_i$), umfassend die mechanischen Eigenschaften nach der Produktion in dem Walzwerk (1), zu jedem der weiteren Walzgüter (2'),
- wobei in einem ersten Schritt (S1') in einer oder mehreren aufeinanderfolgenden Iterationen mit Hilfe des physikalischen Produktionsmodells (12) zu jedem der Produktionsdatensätzen ($P_i$) der weiteren Walzgüter (2') der korrespondierende erste mechanische, weitere Produktions- und metallurgische Datensatz ($C_i$, $F_i$, $K_i$) ermittelt wird,

-- wobei nach jeder Iteration für alle in der zuletzt durchgeführten Iteration ermittelten ersten mechanischen Datensätze ($C_i$) mittels einer ersten Maßfunktion ($\varepsilon_1$) komponentenweise ein Maß für die Abweichung von den korrespondierenden Beprobungsdatensätzen ($B_i$) der weiteren Walzgüter (2') ermittelt wird,
-- wobei in dem Fall, in dem der Wert der ersten Maßfunktion ($\varepsilon_1$) für zumindest eine mechanische Eigenschaft in den ersten mechanischen Datensätzen ($C_i$) der weiteren Walzgüter (2') größer als eine Schranke ($\varepsilon_{max}$) ist, die metallurgischen Modellparameter (y) variiert und in einer weiteren Iteration die ersten mechanischen, die weiteren Produktions- und die metallurgischen Datensätze ($C_i$, $F_i$, $K_i$) der weiteren Walzgüter (2') anhand des physikalischen Produktionsmodells (12) mit den geänderten metallurgischen Modellparametern (y) erneut ermittelt werden,
-- und wobei in dem Fall, in dem der Wert der ersten Maßfunktion ($\varepsilon_1$) für alle mechanischen Eigenschaften in den ersten mechanischen Datensätzen ($C_i$) der weiteren Walzgüter (2') kleiner oder gleich der Schranke ($\varepsilon_{max}$) ist, die Produktionsdatensätze ($P_i$) zusammen mit den in der zuletzt durchgeführten Iteration ermittelten weiteren Produktions- und metallurgischen Datensätze ($F_i$, $K_i$) der weiteren Walzgüter (2') dem statistischen Datenmodell (14) als Eingangsgrößen und die dazu korrespondierenden Beprobungsdatensätze ($B_i$) als Zielgrößen übermittelt werden,

- wobei in einem zweiten Schritt (S2') das statistische Datenmodell (14) anhand der übermittelten Eingangs- und Zielgrößen mit Hilfe eines ersten maschinellen Lernverfahrens ($V_1$) trainiert wird, und
- wobei in einem dritten Schritt (S3') das trainierte statistische Datenmodell (14) anhand von Produktionsdatensätzen ($P_i$) und Beprobungsdatensätzen ($B_i$) von Walzgütern (2") aus einer zweiten, zur ersten Menge ($I_1$) disjunkten zweiten Menge ($I_2$) validiert wird, indem die zu den Walzgütern (2") der zweiten Menge ($I_2$) mittels des physikalischen Produktionsmodells (12) ermittelten weiteren Produktionsdatensätze ($F_i$) und metallurgischen Datensätze ($K_i$) zusammen mit den Produktionsdatensätzen ($P_i$) der Walzgüter (2") der zweiten Menge ($I_2$) dem trainierten statistischen Datenmodell (14) als Eingangsgrößen zugeführt und mittels des trainierten statistischen Datenmodells (14) dazu korrespondierende zweite mechanische Datensätze ($S_i$) ermittelt werden und für diese anhand einer zweiten Maßfunktion ($\varepsilon_2$) komponentenweise ein Maß für eine Abweichung zu den dazu korrespondierenden Beprobungsdatensätzen ($B_i$) ermittelt wird.

10. Verfahren nach Anspruch 9, wobei die mechanischen Eigenschaften ($J_f$) des ersten Walzguts (2) und die Beprobungs-, die ersten und die zweiten mechanischen Datensätze ($B_i$, $C_i$, $S_i$) der weiteren Walzgüter (2') jeweils eine Zugfestigkeit ($Y_s$) und/oder eine Streckgrenze ($T_s$) und/oder eine Bruchdehnung ($A_l$) umfassen.

**11.** Verfahren nach Anspruch 9 oder 10, wobei vor dem ersten Schritt (S1') der zu dem Produktionsdatensatz ($P_i$) korrespondierende Beprobungsdatensatz ($B_i$) von jedem der weiteren Walzgüter (2') auf eine normierte Probengeometrie gemäß der Formel von Oliver transformiert wird.

**12.** Verfahren nach einem der Ansprüche 9 bis 11, wobei die mechanischen Eigenschaften ($J_f$) des ersten Walzguts (2) und die ersten und die zweiten mechanischen Datensätze ($C_i$, $S_i$) der weiteren Walzgüter (2') jeweils für zumindest einen Walzgutabschnitt (20, 20') des ersten und der weiteren Walzgüter (2, 2') ermittelt werden.

**13.** Verfahren nach Anspruch 12, wobei das Walzwerk (1) (N) Walzgerüste ($R_1$,...,$R_N$), (L) Kühlvorrichtungen ($Q_1$,...,$Q_L$), (Z) Walzwerksabschnitte ($A_1$,...,$A_Z$) und zumindest eine Haspelvorrichtung (26) aufweist, und wobei die Produktionsdatensätze ($P_f$, $P_i$) des ersten und der weiteren Walzgüter (2, 2') jeweils zumindest eine der folgenden Stell- oder Messgrößen für zumindest einen Walzgutabschnitt (20, 20') des ersten und der weiteren Walzgüter (2, 2') umfassen:

- einen oder mehrere Fremdelementanteile in dem Walzgutabschnitt (20, 20'),
- eine Dicke und/oder eine Breite des Walzgutabschnitts (20, 20') vor dem Eintritt in ein erstes Walzgerüst ($R_1$) des Walzwerks (1),
- eine Dicke und/oder eine Breite des Walzgutabschnitts (20, 20') nach dem Austritt aus einem letzten Walzgerüst ($R_N$) des Walzwerks (1),
- zumindest eine messtechnisch erfasste Temperatur des Walzgutabschnitts,
- zumindest einen Walzspaltwert eines der Walzgerüste ($R_1$,...,$R_N$) beim Durchtritt des Walzgutabschnitts (20, 20') durch das jeweilige Walzgerüst ($R_1$,...,$R_N$),
- zumindest einen von einer der Kühlvorrichtungen ($Q_1$,...,$Q_L$) abgegebenen Kühlmittelstrom beim Durchtritt des Walzgutabschnitts (20, 20') durch den Wirkbereich der jeweiligen Kühlvorrichtung ($Q_1$,...,$Q_L$), oder
- zumindest eine Geschwindigkeit des Walzgutabschnitts (20, 20') in einem der Walzwerksabschnitte ($A_1$,...,$A_Z$).

**14.** Verfahren nach Anspruch 12 oder 13, wobei der weitere Produktionsdatensatz ($F_i$) für zumindest einen Walzgutabschnitt (20') jedes der weiteren Walzgüter (2') zumindest eine modellierte Kühlrate ($q_z$) und/oder zumindest einen modellierten Temperaturwert ($T_z$) des Walzgutabschnitts (20') beim Passieren von zumindest einem der Walzwerksabschnitte ($A_1$,...,$A_Z$) des Walzwerks (1) umfasst.

**15.** Verfahren nach einem der Ansprüche 12 bis 14, wobei die Datensätze ($K_i$) der weiteren Walzgüter (2') Phasenanteile ($p_k$) und/oder morphologische Kenngrößen ($a_k$) verschiedener metallurgischer Phasen zumindest eines Walzgutabschnitts (20') jedes der weiteren Walzgüter (2') umfassen.

**Claims**

**1.** Method for determining mechanical properties ($J_f$) of a first rolled material (2), produced in a rolling mill (1), using a hybrid model (10), on the basis of a production dataset ($P_f$) of the first rolled material (2), the hybrid model (10) comprising

- production datasets ($P_i$) of further rolled materials (2'), produced in the rolling mill (1), from a first quantity ($I_1$) that each have available corresponding sampling datasets ($B_i$), comprising the mechanical properties of the further rolled materials (2') ascertained by means of a sampling device (9) after production in the rolling mill (1),
- a physical production model (12) comprising a set of metallurgical model parameters (y), for ascertaining

-- a first mechanical dataset ($C_f$), comprising the mechanical properties ($J_f$) of the first rolled material (2) after production in the rolling mill (1),
-- a further production dataset ($F_f$), comprising further production data of the first rolled material (2) during production in the rolling mill (1),
-- and a metallurgical dataset ($K_f$), comprising metallurgical properties of the first rolled material (2) during production in the rolling mill (1), and

- a trained and validated statistical data model (14) for ascertaining a second mechanical dataset ($S_f$), comprising the mechanical properties of the first rolled material (2) after production in the rolling mill (1),

wherein the method comprises a first, a second, a third and a fourth step (S1, S2, S3, S4), wherein

- the first step (S1) involves ascertaining the first mechanical dataset ($C_f$), the further production dataset ($F_f$) and the metallurgical dataset ($K_f$) of the first rolled material (2) from the production dataset ($P_f$) of the first rolled material (2) by means of the physical production model (12),
- the second step (S2) involves ascertaining the second mechanical dataset ($S_f$) of the first rolled material (2) from the production dataset ($P_f$), the further production dataset ($F_f$) and the metallurgical dataset ($K_f$) of the first rolled material (2) by means of the statistical data model (14),
- the third step (S3) involves determining for the production dataset ($P_f$) of the first rolled material (2) an averaged normalized distance value ($d_{f,m}$) in relation to a number (n) of production datasets ($P_i$) of the further rolled materials (2'), and
- the fourth step (S4) involves using the averaged normalized distance value ($d_{f,m}$) to ascertain the mechanical properties ($J_f$) of the first rolled material (2) as a weighted average from the first and second mechanical datasets ($C_f$, $S_f$), the quotients of the mutually corresponding values of the second and first mechanical datasets ($S_f$, $C_f$) being weighted in proportion to the averaged normalized distance value ($d_{f,m}$).

2. Method according to Claim 1, wherein the mechanical properties ($J_f$) of the first rolled material (2) comprise a tensile strength ($Y_s$) and/or a yield strength ($T_s$) and/or an elongation at break ($A_l$).

3. Method according to Claim 1 or 2, wherein the averaged normalized distance value ($d_{f,m}$) is formed from (n) normalized distance values ($d_{f,i}$) of the production dataset ($P_f$) of the first rolled material (2) in relation to a respective production dataset ($P_i$) of one of the further rolled materials (2'), and wherein the number (n) comprises 0.1 to 0.5% of the training datasets of the statistical data model (14).

4. Method according to one of the preceding claims, wherein the mechanical properties ($J_f$) of the first rolled material (2) are ascertained for at least one rolled material section (20) of the first rolled material (2).

5. Method according to Claim 4, the rolling mill (1) having (N) roll stands ($R_1$,...,$R_N$), (L) cooling devices ($Q_1$,...,$Q_L$), (Z) rolling mill sections ($A_1$,...,$A_Z$) and at least one coiling device (26), wherein the production datasets ($P_f$, $P_i$) of the first rolled material and the further rolled materials (2, 2') each comprise at least one of the following manipulated or measured variables for at least one rolled material section (20, 20') of the first rolled material and the further rolled materials (2, 2'):

- one or more foreign element fractions in the rolled material section (20, 20'),
- a thickness and/or a width of the rolled material section (20, 20') before entering a first roll stand ($R_1$),
- a thickness and/or a width of the rolled material section (20, 20') after exiting a last roll stand ($R_N$),
- at least one temperature of the rolled material section (20, 20') recorded by measuring means,
- at least one roll gap value of one of the roll stands ($R_1$,...,$R_N$) when the rolled material section (20, 20') passes through the applicable roll stand ($R_1$,...,$R_N$),
- at least one coolant stream emitted by one of the cooling devices ($Q_1$,...,$Q_L$) when the rolled material section (20, 20') passes through the operating range of the applicable cooling device ($Q_1$,...,$Q_L$), or
- at least one speed of the rolled material section (20, 20') in one of the rolling mill sections ($A_1$,...,$A_Z$).

6. Method according to Claim 4 or 5, wherein the further production dataset ($F_f$) for at least one rolled material section (20) of the first rolled material (2) comprises at least one modeled cooling rate ($q_z$) and/or at least one modeled temperature value ($T_z$) of the rolled material section when passing through at least one of the rolling mill sections ($A_1$,...,$A_Z$) of the rolling mill (1) .

7. Method according to one of Claims 4 to 6, wherein the metallurgical properties of the metallurgical dataset ($K_f$) comprise phase fractions ($p_k$) and/or morphological characteristics ($a_k$) of different metallurgical phases of at least one rolled material section (20) of the first rolled material (2).

8. Method according to one of Claims 4 to 7, wherein a fifth step (S5) involves subjecting the ascertained mechanical properties ($J_f$) of the first rolled material (2) to a cooling correction in which carbide and nitride precipitations in the first rolled material (2) are taken into account during cooling after production in the rolling mill (1).

9. Method for creating a hybrid model (10) for determining mechanical properties ($J_f$) of a first rolled material (2) produced in a rolling mill (1), the hybrid model (10) comprising

- production datasets ($P_i$) of further rolled materials (2'), produced in the rolling mill (1), from a first quantity ($I_1$)

that each have available corresponding sampling datasets ($B_i$), comprising the mechanical properties of the further rolled materials (2') ascertained by means of a sampling device (9) after production in the rolling mill (1),
- a physical production model (12) having a set of metallurgical model parameters (y) for ascertaining

    -- a first mechanical dataset ($C_i$), comprising the mechanical properties after production in the rolling mill (1),
    -- a further production dataset ($F_i$), comprising further production data during production in the rolling mill (1),
    -- and a metallurgical dataset ($K_i$), comprising metallurgical properties during production in the rolling mill (1),
    for each of the further rolled materials (2'), and

    - a statistical data model (14) for ascertaining a second mechanical dataset ($S_i$), comprising the mechanical properties after production in the rolling mill (1), for each of the further rolled materials (2'),
    - wherein one or more consecutive iterations of a first step (S1') involve using the physical production model (12) to ascertain for each of the production datasets ($P_i$) of the further rolled materials (2') the corresponding first mechanical dataset and further production and metallurgical datasets ($C_i$, $F_i$, $K_i$),

        -- wherein each iteration is followed by a first measurement function ($\varepsilon_1$) being used for component-by-component ascertainment, for all first mechanical datasets ($C_i$) ascertained in the most recently performed iteration, of a measure of the deviation from the corresponding sampling datasets ($B_i$) of the further rolled materials (2'),
        -- wherein if the value of the first measurement function ($\varepsilon_1$) for at least one mechanical property in the first mechanical datasets ($C_i$) of the further rolled materials (2') is greater than a limit ($\varepsilon_{max}$), the metallurgical model parameters (y) are varied and, in a further iteration, the first mechanical datasets, the further production datasets and the metallurgical datasets ($C_i$, $F_i$, $K_i$) of the further rolled materials (2') are re-ascertained on the basis of the physical production model (12) with the modified metallurgical model parameters (y),
        -- and wherein if the value of the first measurement function ($\varepsilon_1$) for all mechanical properties in the first mechanical datasets ($C_i$) of the further rolled materials (2') is less than or equal to the limit ($\varepsilon_{max}$), the production datasets ($P_i$) together with the further production and metallurgical datasets ($F_i$, $K_i$) of the further rolled materials (2') ascertained in the most recently performed iteration are transferred to the statistical data model (14) as input variables and the corresponding sampling datasets ($B_i$) as target variables,

    - wherein a second step (S2') involves training the statistical data model (14) on the basis of the transferred input and target variables using a first machine learning method ($V_1$), and
    - wherein a third step (S3') involves validating the trained statistical data model (14) on the basis of production datasets ($P_i$) and sampling datasets ($B_i$) of rolled materials (2") from a second quantity ($I_2$), which is disjunct from the first quantity ($I_1$), by supplying the further production datasets ($F_i$) and metallurgical datasets ($K_i$) ascertained for the rolled materials (2") of the second quantity ($I_2$) by means of the physical production model (12), together with the production datasets ($P_i$) of the rolled materials (2") of the second quantity ($I_2$), to the trained statistical data model (14) as input variables and ascertaining corresponding second mechanical datasets ($S_i$) by means of the trained statistical data model (14) and ascertaining component-by-component a measure of a deviation from the corresponding sampling datasets ($B_i$) for said second mechanical datasets on the basis of a second measurement function ($\varepsilon_2$).

10. Method according to Claim 9, wherein the mechanical properties ($J_f$) of the first rolled material (2) and the sampling datasets, the first and the second mechanical datasets ($B_i$, $C_i$, $S_i$) of the further rolled materials (2') each comprise a tensile strength ($Y_s$) and/or a yield strength ($T_s$) and/or an elongation at break ($A_l$).

11. Method according to Claim 9 or 10, wherein the first step (S1') is preceded by the sampling dataset ($B_i$) that corresponds to the production dataset ($P_i$) being transformed from each of the further rolled materials (2') to a normalized sample geometry according to Oliver's formula.

12. Method according to one of Claims 9 to 11, wherein the mechanical properties ($J_f$) of the first rolled material (2) and the first and second mechanical datasets ($C_i$, $S_i$) of the further rolled materials (2') are each ascertained for at least one rolled material section (20, 20') of the first rolled material and the further rolled materials (2, 2').

13. Method according to Claim 12, wherein the rolling mill (1) has (N) roll stands ($R_1$,...,$R_N$), (L) cooling devices ($Q_1$,...,$Q_L$), (Z) rolling mill sections ($A_1$,...,$A_Z$) and at least one coiling device (26), and wherein the production datasets ($P_f$, $P_i$) of the first rolled material and the further rolled materials (2, 2') each comprise at least one of the following manipulated or measured variables for at least one rolled material section (20, 20') of the first rolled material and the further rolled materials (2, 2'):

- one or more foreign element fractions in the rolled material section (20, 20'),
- a thickness and/or a width of the rolled material section (20, 20') before entering a first roll stand ($R_1$) of the rolling mill (1),
- a thickness and/or a width of the rolled material section (20, 20') after exiting a last roll stand ($R_N$) of the rolling mill (1),
- at least one temperature of the rolled material section recorded by measuring means,
- at least one roll gap value of one of the roll stands ($R_1,...,R_N$) when the rolled material section (20, 20') passes through the respective roll stand ($R_1,...,R_N$),
- at least one coolant stream emitted by one of the cooling devices ($Q_1,...,Q_L$) when the rolled material section (20, 20') passes through the operating range of the respective cooling device ($Q_1,...,Q_L$), or
- at least one speed of the rolled material section (20, 20') in one of the rolling mill sections ($A_1,..., A_Z$).

14. Method according to Claim 12 or 13, wherein the further production dataset ($F_i$) for at least one rolled material section (20') of each of the further rolled materials (2') comprises at least one modeled cooling rate ($q_z$) and/or at least one modeled temperature value ($T_z$) of the rolled material section (20') when passing through at least one of the rolling mill sections ($A_1,...,A_Z$) of the rolling mill (1) .

15. Method according to one of Claims 12 to 14, wherein the datasets ($K_i$) of the further rolled materials (2') comprise phase fractions ($p_k$) and/or morphological characteristics ($a_k$) of different metallurgical phases of at least one rolled material section (20') of each of the further rolled materials (2').

## Revendications

1. Procédé de détermination des propriétés mécaniques ($J_f$) d'un premier produit laminé (2) produit dans un laminoir (1) à l'aide d'un modèle hybride (10), à partir d'un jeu de données de production ($P_f$) du premier produit laminé (2), le modèle hybride (10) comprenant

   des jeux de données de production ($P_i$) d'autres produits laminés (2') produits dans le laminoir (1) à partir d'une première quantité ($I_1$) pour lesquels des jeux de données d'échantillonnage ($B_i$) correspondant respectivement, comprenant les propriétés mécaniques déterminées des autres produits laminés (2') après la production dans le laminoir (1) au moyen d'un dispositif d'échantillonnage (9) sont disponibles

   - un modèle de production physique (12) comprenant un jeu de paramètres de modèles métallurgiques ($\gamma$), pour déterminer

     -- un premier jeu de données mécaniques ($C_f$), comprenant les propriétés mécaniques ($J_f$) du premier produit laminé (2) après la production dans le laminoir (1),
     -- un autre jeu de données de production ($F_f$), comprenant d'autres données de production du premier produit laminé (2) pendant la production dans le laminoir (1),
     -- et un jeu de données métallurgiques ($K_f$), comprenant des propriétés métallurgiques du premier produit laminé (2) pendant la production dans le laminoir (1), et

   - un modèle de données statistiques (14) entraîné et validé pour déterminer un deuxième jeu de données mécaniques ($S_f$), comprenant les propriétés mécaniques du premier produit laminé (2) après la production dans le laminoir (1),

   le procédé comprenant de première, deuxième, troisième et quatrième étapes (S1, S2, S3, S4), dans lesquelles

   - dans la première étape (S1), est déterminé à partir du jeu de données de production ($P_f$) du premier produit laminé (2), au moyen du modèle de production physique (12), le premier jeu de données mécaniques ($C_f$), l'autre jeu de données de production ($F_f$) et le jeu de données métallurgiques ($K_f$) du premier produit laminé (2),
   - dans la deuxième étape (S2), est déterminé à partir du jeu de données de production ($P_f$), du jeu de données de production supplémentaire ($F_f$) et du jeu de données métallurgiques ($K_f$) du premier produit laminé (2) au moyen du modèle de données statistiques (14) le deuxième jeu de données mécaniques ($S_f$)

du premier produit laminé (2),

- dans la troisième étape (S3), est déterminée pour le jeu de données de production ($P_f$) du premier produit laminé (2) une valeur de distance normalisée moyenne ($d_{f,m}$) par rapport à un nombre (n) de jeux de données de production ($P_i$) des autres produits laminés (2'), et

- dans la quatrième étape (S4), à l'aide de la valeur de distance normalisée moyenne ($d_{f,m}$), sont déterminés les propriétés mécaniques ($J_f$) du premier produit laminé (2) comme moyenne pondérée à partir du premier et du deuxième jeu de données mécaniques ($C_f$, $S_f$), les quotients des valeurs correspondant les unes aux autres du deuxième et du premier jeu de données mécaniques ($S_f$, $C_f$) étant pondérés proportionnellement à la valeur de distance normalisée moyenne ($d_{f,m}$).

2. Procédé selon la revendication 1, dans lequel les propriétés mécaniques ($J_f$) du premier produit laminé (2) comprennent une résistance à la traction ($Y_s$) et/ou une limite d'élasticité ($T_s$) et/ou un allongement à la rupture ($A_1$).

3. Procédé selon la revendication 1 ou 2, dans lequel la valeur de distance normalisée moyenne ($d_{f,m}$) est formée à partir de (n) valeurs de distance normalisées ($d_{f,i}$) du jeu de données de production ($P_f$) du premier produit laminé (2) par rapport respectivement à un jeu de données de production ($P_i$) de l'un des autres produits laminés (2'), et dans lequel le nombre (n) comprend de 0,1 à 0,5 pour cent des jeux de données d'apprentissage du modèle de données statistiques(14).

4. Procédé selon l'une des revendications précédentes, dans lequel les propriétés mécaniques ($J_f$) du premier produit laminé (2) sont déterminées pour au moins un tronçon de produit laminé (20) du premier produit laminé (2).

5. Procédé selon la revendication 4, le laminoir (1) présentant (N) cadres de laminoir ($R_1$, ..., $R_N$), (L) dispositifs de refroidissement ($Q_1$, ..., $Q_L$), (Z) sections de laminoir ($A_1$, ..., $A_Z$) et au moins un dispositif de dévidage (26), dans lequel les jeux de données de production ($P_f$, $P_i$) du premier et des autres produits laminés (2, 2') comprennent respectivement au moins l'une des variables de réglage ou de mesure suivantes pour au moins un tronçon de produit laminé (20, 20') du premier et des autres produits laminés (2, 2') :

- une ou plusieurs parties d'éléments étrangers dans le tronçon de produit laminé (20, 20'),
- une épaisseur et/ou une largeur du tronçon de produit laminé (20, 20') avant l'entrée dans un premier cadre de laminoir ($R_1$),
- une épaisseur et/ou une largeur du tronçon de produit laminé (20, 20') après la sortie d'un dernier cadre de laminoir ($R_N$),
- au moins une température du tronçon de produit laminé (20, 20') détectée par une technique de mesure,
- au moins une valeur d'écartement de laminage de l'un des cadres de laminage ($R_1$, ..., $R_N$) lors du passage du tronçon de produit laminé (20, 20') à travers le cadre de laminage correspondant ($R_1$, ..., $R_N$),
- au moins un flux d'agent de refroidissement délivré par l'un des dispositifs de refroidissement ($Q_1$, ..., $Q_L$) lors du passage du tronçon de produit laminé (20, 20') à travers la zone active du dispositif de refroidissement correspondant ($Q_1$, ..., $Q_L$), ou
- au moins une vitesse du tronçon de produit laminé (20, 20') dans l'une des sections de laminoir ($A_1$, ..., $A_Z$).

6. Procédé selon la revendication 4 ou 5, dans lequel l'autre jeu de données de production ($F_f$) comprend, pour au moins un tronçon de produit laminé (20) du premier produit laminé (2), au moins une vitesse de refroidissement modélisée ($q_z$) et/ou au moins une valeur de température modélisée ($T_z$) du tronçon de produit laminé lors du passage par au moins l'une des sections de laminoir ($A_1$, ..., $A_Z$) du laminoir (1).

7. Procédé selon l'une des revendications 4 à 6, dans lequel les propriétés métallurgiques du jeu de données métallurgiques ($K_f$) comprennent des proportions de phases ($p_k$) et/ou des caractéristiques morphologiques ($a_k$) de différentes phases métallurgiques d'au moins un tronçon de produit laminé (20) du premier produit laminé (2).

8. Procédé selon l'une des revendications 4 à 7, dans lequel, dans une cinquième étape (S5), les propriétés mécaniques déterminées ($J_f$) du premier produit laminé (2) sont soumises à une correction de refroidissement tenant compte de la précipitation des carbures et des nitrures dans le premier produit laminé (2) pendant le refroidissement après la production dans le laminoir (1).

9. Procédé de production d'un modèle hybride (10) pour la détermination de propriétés mécaniques ($J_f$) d'un premier produit laminé (2) produit dans un laminoir (1), le modèle hybride (10) comprenant

- des jeux de données de production ($P_i$) d'autres produits laminés (2') produits dans le laminoir (1) à partir d'une première quantité ($I_1$) pour lesquels des jeux de données d'échantillonnage ($B_i$) correspondant respectivement, comprenant les propriétés mécaniques déterminées des autres produits laminés (2') après la production dans le laminoir (1) au moyen d'un dispositif d'échantillonnage (9) sont disponibles

- un modèle de production physique (12) avec un jeu de paramètres de modèle métallurgique ($\gamma$) pour déterminer

-- un premier jeu de données mécaniques ($C_i$), comprenant les propriétés mécaniques après la production dans le laminoir (1),

-- un autre jeu de données de production ($F_i$), comprenant d'autres données de production pendant la production dans le laminoir (1),

-- et un jeu de données métallurgiques ($K_i$) comprenant des propriétés métallurgiques pendant la production dans le laminoir (1),

pour chacun des autres produits laminés (2'), et

- un modèle de données statistiques (14) pour déterminer un deuxième jeu de données mécaniques ($S_i$), comprenant les propriétés mécaniques après la production dans le laminoir (1), pour chacun des autres produits laminés (2'),

- dans lequel, dans une première étape (S1'), est déterminé, en une ou plusieurs itérations successives, à l'aide du modèle de production physique (12), pour chacun des jeux de données de production ($P_i$) des autres produits laminés (2'), le premier autre jeu de données mécaniques, de production et métallurgiques ($C_i$, $F_i$, $K_i$) correspondant,

-- dans lequel, après chaque itération pour tous les premiers jeux de données mécaniques ($C_i$) déterminés dans la dernière itération effectuée, une mesure de l'écart par rapport aux jeux de données d'échantillonnage correspondants ($B_i$) des autres produits laminés (2') est déterminée moyennant une première fonction de mesure ($\varepsilon_1$) composante par composante,

-- dans lequel, dans le cas où la valeur de la première fonction de mesure ($\varepsilon_1$) pour au moins une propriété mécanique dans les premiers jeux de données mécaniques ($C_i$) des autres produits laminés (2') est supérieure à une limite ($\varepsilon_{max}$), on fait varier les paramètres de modèle métallurgique ($\gamma$) et, dans une autre itération, les autres jeux de données de production et les jeux de données métallurgiques ($C_i$, $F_i$, $K_i$) des autres produits laminés (2') sont à nouveau déterminés à l'aide du modèle de production physique (12) avec les paramètres de modèle métallurgique modifiés ($\gamma$),

-- et où, dans le cas où la valeur de la première fonction de mesure ($\varepsilon_1$) pour toutes les propriétés mécaniques dans les premiers jeux de données mécaniques ($C_i$) des autres produits laminés (2') est inférieure ou égale à la limite ($\varepsilon_{max}$), les jeux de données de production ($P_i$) sont transmis, conjointement aux autres jeux de données de production et métallurgiques ($F_i$, $K_i$) des autres produits laminés (2') déterminés dans la dernière itération effectuée, au modèle de données statistiques (14) en tant que variables d'entrée et les jeux de données d'échantillonnage ($B_i$) correspondants en tant que variables cibles,

- dans lequel, dans une deuxième étape (S2'), le modèle de données statistiques (14) est entraîné à l'aide des variables d'entrée et des variables cibles transmises à l'aide d'un premier procédé d'apprentissage automatique ($V_1$), et

- dans lequel, dans une troisième étape (S3'), le modèle de données statistiques (14) entraîné est validé à l'aide de jeux de données de production ($P_i$) et de jeux de données d'échantillonnage ($B_i$) de produits laminés (2") d'une deuxième quantité ($I_2$) séparée de la première quantité ($I_1$), en ce que les autres jeux de données de production ($F_i$) déterminés pour les produits laminés (2") de la deuxième quantité ($I_2$) au moyen du modèle de production physique (12) et les jeux de données métallurgiques ($K_i$) sont transmis conjointement avec les jeux de données de production ($P_i$) des produits laminés (2") de la deuxième quantité ($I_2$) au modèle de données statistiques entraîné (14) en tant que variables d'entrées et au moyen du modèle de données statistiques entraîné (14), sont déterminés de deuxièmes jeux de données mécaniques correspondants ($S_i$) et que pour ceux-ci, à l'aide d'une deuxième fonction de mesure ($\varepsilon_2$), est déterminée, composante par composante, une mesure d'un écart par rapport aux jeux de données d'échantillonnage correspondants ($B_i$).

**10.** Procédé selon la revendication 9, dans lequel les propriétés mécaniques ($J_f$) du premier produit laminé (2) et les jeux de données d'échantillonnage, les premiers et les seconds jeux de données mécaniques ($B_i$, $C_i$, $S_i$) des autres produits laminés (2') comprennent chacun une résistance à la traction ($Y_s$) et/ou une limite d'élasticité ($T_s$) et/ou un allongement à la rupture ($A_1$).

**11.** Procédé selon la revendication 9 ou 10, dans lequel, avant la première étape (S1'), le jeu de données d'échantillonnage ($B_i$) correspondant au jeu de données de production ($P_i$) de chacun des autres produits laminés (2') est transformé en une géométrie d'échantillon normalisée selon la formule d'Oliver.

**12.** Procédé selon l'une des revendications 9 à 11, dans lequel les propriétés mécaniques ($J_f$) du premier produit laminé (2) et les premiers et deuxièmes jeux de données mécaniques ($C_i$, $S_i$) des autres produits laminés (2') sont déterminés respectivement pour au moins un tronçon de produit laminé (20, 20') du premier et des autres produits laminés (2, 2').

**13.** Procédé selon la revendication 12, dans lequel le laminoir (1) comprend (N) cadres de laminoir ($R_1$, ..., $R_N$), (L) dispositifs de refroidissement ($Q_1$, ..., $Q_L$), (Z) sections de laminoir ($A_1$, ..., $A_Z$) et au moins un dispositif de dévidage (26), et dans lequel les jeux de données de production ($P_f$, $P_i$) du premier et des autres produits laminés (2, 2') comprennent respectivement au moins l'une des variables de réglage ou de mesure suivantes pour au moins un tronçon de produit laminé (20, 20') du premier et des autres produits laminés (2, 2') :

   - une ou plusieurs parties d'éléments étrangers dans le tronçon de produit laminé (20, 20'),
   - une épaisseur et/ou une largeur du tronçon de produit laminé (20, 20') avant l'entrée dans un premier cadre de laminoir ($R_1$) du laminoir (1),
   - une épaisseur et/ou une largeur du tronçon de produit laminé (20, 20') après la sortie d'un dernier cadre de laminoir ($R_N$) du laminoir (1),
   - au moins une température mesurée du tronçon de produit laminé,
   - au moins une valeur d'écartement de laminage de l'un des cadres de laminage ($R_1$, ..., $R_N$) lors du passage du tronçon de produit laminé (20, 20') à travers le cadre de laminage respectif ($R_1$, ..., $R_N$),
   - au moins un flux d'agent de refroidissement délivré par l'un des dispositifs de refroidissement ($Q_1$, ..., $Q_L$) lors du passage du tronçon de produit laminé (20, 20') à travers la zone active du dispositif de refroidissement respectif ($Q_1$, ..., $Q_L$), ou
   - au moins une vitesse du tronçon de produit laminé (20, 20') dans l'une des sections de laminoir ($A_1$, ..., $A_Z$).

**14.** Procédé selon la revendication 12 ou 13, dans lequel l'autre jeu de données de production ($F_i$) comprend, pour au moins un tronçon de produit laminé (20') de chacun des autres produits laminés (2'), au moins une vitesse de refroidissement modélisée ($q_z$) et/ou au moins une valeur de température modélisée ($T_z$) du tronçon de produit laminé (20') lors du passage dans au moins l'une des sections de laminoir ($A_1$, ..., $A_Z$) du laminoir (1).

**15.** Procédé selon l'une des revendications 12 à 14, dans lequel les jeux de données ($K_i$) des autres produits laminés (2') comprennent des proportions de phases ($p_k$) et/ou des caractéristiques morphologiques ($a_k$) de différentes phases métallurgiques d'au moins un tronçon de produit laminé (20') de chacun des autres produits laminés (2').

FIG 1

EP 4 124 398 B1

FIG 2

$J_f$: $\{Y_S, T_S, A_l\}$

FIG 3

**FIG 4**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- CN 101046682 A
- CN 107609647 A
- JP 2005315703 A
- US 20180260717 A1
- JP 2011220708 A

### In der Beschreibung aufgeführte Nicht-Patentliteratur

- **WOLFF et al.** *Archives of Mechanics,* 2007, vol. 59 (4-5), 435-466
- **BUCHMAYR.** Werkstoff- und Produktionstechnik mit Mathcad. Springer-Verlag, 2002, 87
- **BUCHMAYR.** Werkstoff- und Produktionstechnik mit Mathcad. Springer-Verlag, 2002, 122-123
- **IBABE.** Thin Slab Direct Rolling of Microalloyed Steels. *Materials Science Foundations,* vol. 33, ISSN 1422-3597, ISBN 0-87849-485-5
- **LERNENS FRIEDMAN et al.** The elements of statistical learning. *Springer series in statistics,* 2001, ISBN 0-387-95284-5
- **HANLON et al.** Critical Assessment 10: Tensile elongation of strong automotive steels as function of testpiece geometry. *Materials Science and Technology,* 2015, vol. 31 (4), 385-388